# FASCICULE DE BREVET EUROPEEN

(11) **EP 2 331 673 B1**
(45) Date de publication et mention de la délivrance du brevet: **28.10.2020**
(21) Numéro de dépôt: 09752400.3
(22) Date de dépôt: 12.10.2009
(51) Int. Cl.: C12M 3/00

(54) **DISPOSITIF DE CULTURE CELLULAIRE**
VORRICHTUNG ZUR ZELLKULTUR
DEVICE FOR CELL CULTURE

(30) Priorité: 10.10.2008 FR 0805606
(43) Date de publication de la demande: 15.06.2011
(73) Titulaire: CNRS-DAE, 75016 Paris (FR); Institut Curie, 75248 Paris Cedex 05 (FR); Sorbonne Université, 75006 Paris (FR)
(72) Inventeur: VIOVY Jean-Louis, F-75013 PARIS (FR); PEYRIN Jean-Michel, F-75010 PARIS (FR); BRUGG Bernard, 95000 CERGY (FR); SAIAS Laure, F-75005 PARIS (FR); GOUGIS Paul, F-75019 PARIS (FR); VIGNES, Maeva, 75015 Paris (FR)
(74) Mandataire: Icosa
(86) Numéro de dépôt international: PCT/FR2009/001198
(87) Numéro de publication internationale: WO 2010/040920

(56) Documents cités:
- EP-A- 0 483 117
- WO-A-2004/029221
- WO-A-2006/037033
- KR-B1- 100 709 284
- US-B2- 7 419 822
- TAYLOR A M ET AL: "Microfluidic Multicompartment Device for Neuroscience Research" LANGMUIR, ACS, WASHINGTON, DC, US, vol. 19, no. 5, 4 décembre 2002 (2002-12-04), pages 1551-1556, XP002989621 ISSN: 0743-7463
- TAYLOR A M ET AL: "A microfluidic culture platform for CNS axonal injury, regeneration and transport" NATURE METHODS, NATURE PUBLISHING GROUP, GB, vol. 2, no. 8, 1 août 2005 (2005-08-01), pages 599-605, XP002544760 ISSN: 1548-7091
- GROSS ET AL: "Applications of microfluidics for neuronal studies" JOURNAL OF NEUROLOGICAL SCIENCES, ELSEVIER SCIENTIFIC PUBLISHING CO, AMSTERDAM, NL, vol. 252, no. 2, 8 janvier 2007 (2007-01-08), pages 135-143, XP005836716 ISSN: 0022-510X
- THIRUKUMARAN T KANAGASABAPATHI ET AL: "IN-VITRO COMPARTMENTED NEUROFLUIDIC SYSTEM FOR STUDYING NEURAL NETWORKS", CONFERENCE PROCEEDINGS OF THE 6TH INTERNATIONAL MEETING ON SUBSTRATE-INTEGRATED MICRO ELECTRODE ARRAYS : JULY 8 - 11, 2008, REUTLINGEN, GERMANY; [BIOPRO EDITION],, 8 July 2008 (2008-07-08), pages 317-318, XP003034067, ISBN: 3-938345-05-5

## Description

La présente invention concerne les dispositifs et procédés de culture cellulaire, notamment de cellules neuronales.

Le cerveau est une structure extrêmement complexe composée de plusieurs aires neuronales connectées entre elles. Les études expérimentales in vivo préservent cette structure globale, mais ne sont pas adaptées à l'étude aux échelles cellulaires.

Les cultures de cellules dissociées permettent de décrire bien plus en détail le système étudié. C'est pourquoi de nombreux laboratoires procèdent à des cultures neuronales. Traditionnellement, ces cultures de neurones sont réalisées dans des boîtes de petri ou des puits de culture. Ces cultures cellulaires trouvent des applications comme modèle réductionniste dans l'étude des maladies neuro-dégénératives (Alzheimer, Huntington, Creutzfeldt-Jakob, etc.) mais aussi en biologie du développement pour la compréhension des mécanismes moléculaires et cellulaires de la différenciation neuronale.

Cependant, dans ces systèmes les connexions neuronales se font aléatoirement et il est impossible d'y reconstituer une architecture semblable à celles que l'on trouve in vivo.

La structure en réseau du système nerveux central (CNS) est complètement absente, et ne permet pas d'étudier comment les différentes couches neuronales interagissent.

Une autre méthode consiste à utiliser des tranches de différentes parties du cerveau cultivées ex vivo.

Même si l'intégrité des couches neuronales est préservée par cette méthode, la complexité des tissus prélevés pose vite problème. Afin de mieux comprendre la propagation de la mort neuronale et les mécanismes de développement dans les différentes couches du cerveau, il convient de développer de nouveaux dispositifs expérimentaux permettant de contrôler l'architecture des réseaux des neurones cultivés in vitro.

La microfluidique est un outil de choix pour la biologie cellulaire et en particulier pour les neurosciences.

Dans WO200434016, Jeon et al.*,* inspirés des travaux de Campenot [Campenot, R. B. Local control of neurite development by nerve growth factor. Proc. Natl. Acad. Sci. USA. 1977, 74(10), 4516-4519], proposent une configuration de circuit microfluidique permettant d'isoler le soma des neurones de leur axone.

Cette configuration est adaptée aux neurones du système nerveux central (CNS).

Le compartiment dit somatique est le canal dans lequel sont introduits les neurones fraîchement disséqués.

Le compartiment distal est celui vers lequel se dirigent les axones en traversant des microcanaux. Le soma des neurones ne peut pas traverser les microcanaux. En effet, les microcanaux sont trop fins pour permettre au soma de traverser.

Ce dispositif est un premier pas vers le contrôle des cultures neuronales du CNS in vitro. Les temps de diffusion dans les microcanaux sont longs, ce qui permet de traiter de façon séparée les compartiments distal et somatique. La diffusion de ce qui est contenu dans l'un des compartiments vers l'autre est compensée en imposant un différentiel de pression. Il suffit pour cela de mettre dans l'un des réservoirs d'un des compartiments un volume de liquide plus grand, pour imposer un différentiel de pression hydrostatique entre différents compartiments.

Cependant, ce dispositif présente encore de nombreuses limitations. D'une part, s'il permet de séparer les compartiments cellulaires, il ne permet pas d'induire de connexion axonale dirigée entre deux populations de neurones, puisque les axones peuvent parcourir les microcanaux dans les deux sens.

De plus, TAYLOR et al., "Microfluidic culture platform for CNS axonal injury, regeneration and transport", NATURE METHOD, 1er août 2005, vol. 2, no. 8, p599-605 concerne un dispositif pour la culture cellulaire de cellules neuronales, mais dans lequel les microcanaux interconnectant les chambres ont une largeur constante.

Enfin, KR100709284 divulgue un dispositif pour la mesure de l'activité chemotactique de cellules en culture, comprenant au moins deux chambres microfluidique interconnectées par un microcanal dont la largeur se réduit d'un côté à l'autre. Cependant, ce dispositif ne comprend pas de macrocanaux.

Les publications WO 2006/037033 et US 7 419822, qui sont incorporés par référence, se rapportent également à des dispositifs de culture cellulaire adaptés à la culture de neurones.

La présente invention vise entre autres à remédier à ces différentes limitations, et de permettre ainsi des études, des procédés et des criblages impossibles avec les dispositifs de l'art actuel.

### Résumé

L'invention a pour objet, selon l'un de ses aspects, un dispositif de culture cellulaire, notamment de cellules neuronales, comportant :
- un support définissant une première chambre microfluidique (2a) destinée à être ensemencée par une première culture cellulaire, et au moins une deuxième chambre microfluidique (2b), et
- un système d'interconnexion fluidique (3) reliant les première et deuxième chambres microfluidiques (2a, 2b) et permettant à des extensions cellulaires, notamment des axones, de s'étendre d'une chambre microfluidique vers l'autre chambre microfluidique, dans lequel la première chambre microfluidique et la deuxième chambre microfluidique comprennent chacune un macrocanal (4a, 4b) relié à ses extrémités à des portions plus larges formant des réservoirs (5), le système d'interconnexion fluidique (3) comprenant au moins un microcanal (10) qui est relié à ses extrémités au macrocanal de la première chambre microfluidique (4a) et au macrocanal de la deuxième chambre microfluidique (4b),

### caractérisé en ce que :

- la largeur dudit au moins un microcanal se réduit en progressant d'une chambre microfluidique vers l'autre, et
- le système d'interconnexion est réalisé de manière à privilégier la progression d'au moins un premier type d'extensions cellulaires par rapport à au moins un second type d'extensions cellulaires, lesdits premier et second types d'extensions cellulaires différant soit par la chambre microfluidique dont ils proviennent, soit par le type cellulaire dont ils sont l'extension.

Dans des exemples de réalisation, les macrocanaux (4a, 4b) des première et deuxième chambres microfluidiques sont parallèles.

Dans des exemples de réalisation, une troisième chambre microfluidique (4c) communique avec le système d'interconnexion fluidique (3).

Dans des exemples de réalisation, le système d'interconnexion (3) comporte un microcanal (10) dont au moins une portion présente une forme trapézoïdale lorsqu'observé de dessus.

Dans des exemples de réalisation, le système d'interconnexion (3) comporte un canal non rectiligne.

Dans des exemples de réalisation, le système d'interconnexion (3) comporte au moins une portion dont la surface a été traitée chimiquement ou biochimiquement de façon à avoir une affinité pour au moins un type de cellule ou un type de comportement cellulaire, en particulier ledit traitement chimique comporte l'exposition à au moins un type de molécule sélectionné parmi la fibronectine, les cadhérines, le collagène, la laminine, la poly-lysine, les molécules comportant des groupements succinimides, le (N-Sulfosuccinimidyl-6-[4'-azido-2'-nitrophenylamino] hexanoate), les molécules chimiques réactives photo-activables.

Dans des exemples de réalisation, le système d'interconnexion (3) comporte des microcanaux (10) d'épaisseur comprise entre 1 et 5 µm, préférentiellement entre 2 et 4 µm.

Dans des exemples de réalisation, le dispositif comporte au moins une chambre microfluidique monocellulaire (80), dimensionnée pour ne contenir que le soma d'une seule cellule, cette chambre microfluidique monocellulaire communiquant avec le système d'interconnexion et avec l'une des première et deuxième chambres microfluidiques

L'invention a encore pour objet, selon un autre de ses aspects, un procédé de culture cellulaire, notamment de cellules neuronales, dans lequel en ensemence avec des cellules au moins une chambre microfluidique (2a, 2b) d'un dispositif tel que défini plus haut, lesdites cellules pouvant, lors de leur multiplication et/ou de leur différenciation, donner lieu à des extensions cellulaires.

L'invention a encore pour objet, selon un autre de ses aspects, l'utilisation du dispositif tel que défini plus haut pour:
(i) une compartimentalisation neuronale efficace, l'imposition d'une directionnalité à la croissance axonale, la sélection entre différents types d'axones, et/ou la création de réseaux de cellules impliquant différents types de cellules contenus dans des chambres différentes, et présentant des connexions dirigées par l'intermédiaire des extensions cellulaires; ou
(ii) la reconstruction orientée de réseaux neuronaux du plus simples au plus complexe, impliquant éventuellement différents sous-types neuronaux, et/ou une combinaison de neurones et de cellules non-neuronales, et/ou différents types d'interaction axonales et/ou dendritiques entre neurones; ou
(iii) reconstruire et étudier les interactions entre cellules neuronales et cellules non-neuronales; ou
(iv) adresses des compartiments neuronaux spécifiques avec des réactifs et des médicaments, de collecter pour analyse des biomarqueurs provant de certains de ces compartiments.

### Ultérieures informations pour la mise en oeuvre

Le dispositif peut permettre d'induire des connexions cellulaires dirigées entre deux populations de cellules, en particulier des connections axonales dirigées. La deuxième chambre microfluidique (2b) peut être ensemencée avec au moins un deuxième type cellulaire.

Le système d'interconnexion peut comporter une pluralité de canaux, encore appelés microcanaux, et/ou une pluralité de microstructures.

Le système d'interconnexion peut comporter au moins un canal ou un réseau de microstructures présentant une asymétrie entre le côté dudit canal ou dudit réseau connecté à la première chambre microfluidique, et le côté dudit canal ou dudit réseau connecté à la deuxième chambre microfluidique.

Le système d'interconnexion peut comporter au moins un canal dont au moins une dimension se réduit en progressant d'une chambre vers l'autre, ladite au moins une dimension comportant par exemple la largeur du canal. En particulier, le système d'interconnexion peut comporter au moins un canal rétrécissant, encore appelé « diode ». Ladite dimension peut être, à l'endroit du débouché dudit canal dans la première chambre microfluidique ou dans la deuxième chambre microfluidique, inférieure ou égale à 5 µm.

Le système d'interconnexion peut comporter un canal dont au moins une portion présente une forme trapézoïdale lorsqu'observé de dessus. Le canal peut présenter une portion convergente prolongée par une portion de largeur constante.

Le système d'interconnexion peut comporter un canal non rectiligne. En variante, tous les canaux du système d'interconnexion sont rectilignes.

Lesdits canaux peuvent présenter des interconnexions ou des ramifications.

Le réseau de microstructures peut comporter des obstacles empêchant la propagation d'axones en ligne droite.

Les obstacles peuvent être disposés de façon à imposer à tout chemin continu entre la première chambre microfluidique et la deuxième chambre microfluidique au moins une portion dans laquelle le rayon de courbure dudit chemin est inférieur à 20 µm, de préférence inférieur à 10 µm, plus préférentiellement encore inférieur à 7 µm, 5 µm, voire 3 µm.

La première chambre peut être symétrique de l'autre chambre par rapport à un plan de symétrie.

L'écartement entre la première et la deuxième chambre est par exemple compris entre 3 µm et 10000 µm, par exemple entre 10 µm et 10000 µm.

Le système d'interconnexion peut comporter au moins une portion dont la surface a été traitée chimiquement ou biochimiquement de façon à avoir une affinité pour au moins un type de cellule ou un type de comportement cellulaire.

Le traitement chimique peut comporter l'exposition à moins un type de molécule sélectionné parmi la fibronectine, les cadhérines, le collagène, la laminine, les molécules comportant des groupements succinimides, le (N-Sulfosuccinimidyl-6-[4'-azido-2'-nitrophenylamino] hexanoate), les molécules chimiques réactives photo-activables.

Le système d'interconnexion peut comporter des microcanaux ou des microstructures d'épaisseur inférieure à celles des première et seconde chambres microfluidiques, et de préférence comprise entre 1 et 5 µm, plus préférentiellement encore entre 2 et 4 µm.

Dans une variante, la seule communication fluidique de la première ou deuxième chambre avec l'extérieur de cette chambre a lieu par l'intermédiaire du système d'interconnexion. Dans une autre variante, une communication fluidique peut avoir lieu non seulement par l'intermédiaire du système d'interconnexion mais également par l'intermédiaire d'au moins une membrane ne laissant passer que des solutés et non des prolongements cellulaires.

Cette membrane est par exemple située sur le dessus de la chambre. Il peut s'agir par exemple d'une membrane micro perforée en PDMS ou en nitrocellulose.

Dans des exemples de mise en œuvre de l'invention, le système d'interconnexion ne sert pas à filtrer un écoulement, notamment sanguin.

Le dispositif peut comporter au moins une chambre microfluidique monocellulaire, dimensionnée pour ne contenir que le soma d'une seule cellule, cette chambre microfluidique monocellulaire communiquant avec le système d'interconnexion et avec l'une des première et deuxième chambres microfluidiques.

La chambre microfluidique monocellulaire peut avoir une épaisseur plus faible que celle des première et deuxième chambres microfluidiques, et plus élevée que celle dudit système d'interconnexion. La chambre microfluidique monocellulaire peut être plus proche de l'une des chambres que de l'autre.

Le dispositif microfluidique peut comporter au moins trois chambres microfluidiques, à savoir une première et une deuxième chambre reliées par un premier système d'interconnexion. L'un au moins des premier et deuxième systèmes est tel que défini plus haut, à savoir permet à des extensions cellulaires, notamment des axones, de s'étendre d'une chambre vers l'autre chambre, en privilégiant la progression d'au moins un premier type d'extensions cellulaires par rapport à au moins un second type d'extensions cellulaires. Chaque système d'interconnexion peut comporter des ensembles de micro-canaux. Les micro-canaux du deuxième système d'interconnexion peuvent être orientés autrement que dans le prolongement des micro-canaux du premier système d'interconnextion, par exemple sensiblement perpendiculairement à ceux-ci. L'un des avantages de cette configuration est de réduire le risque que des extensions provenant de la première chambre et atteignant la deuxième chambre ne progressent vers la troisième chambre.

Le dispositif peut comporter au moins une chambre microfluidique dont le volume est compris entre 100 et 10 000 µm³, et de préférence entre 500 et 5000 µm³, et communiquant de façon fluidique avec le système d'interconnexion et avec l'une des première et deuxième chambres microfluidiques.

Le système d'interconnexion peut être traversé par au moins un canal reliant par exemple deux régions opposées, disposées de part et d'autre du système d'interconnexion. Un tel canal peut permettre par exemple de réduire le risque de contamination d'une chambre par un composé introduit dans l'autre chambre. Le canal peut aussi permettre de réaliser une axotomie, en y faisant circuler une substance tel qu'un détergent comme par exemple le tritonX ou la saponine. On peut faire circuler dans le canal toute autre molécule pouvant endommager, protéger ou modifier le métabolisme des axones.

Le système d'interconnexion peut être délimité sur au moins une partie de sa surface par une paroi poreuse permettant l'échange avec un compartiment annexe de molécules et d'ions, mais ne permettant pas l'échange, avec ledit compartiment annexe, de cellules.

Le dispositif de culture cellulaire peut comporter en outre au moins une microélectrode, et de préférence un réseau de microélectrodes, reliée à un appareil de mesure permettant d'étudier des processus électrophysiologiques au sein d'au moins une cellule présente dans ledit dispositif.

Le dispositif peut comporter
- des premier et second macrocanaux,
- un microcanal relié à une première extrémité au premier macrocanal,
- une chambre microfluidique monocellulaire communiquant avec le deuxième macrocanal et avec une deuxième extrémité du microcanal, les premier et deuxième macrocanaux communiquant entre eux par l'intermédiaire du microcanal et de la chambre microfluidique monocellulaire, la chambre microfluidique monocellulaire étant dimensionnée de façon de ne pouvoir recevoir que le soma d'une seule cellule.

Le dispositif peut comporter au moins deux systèmes d'interconnexion dont l'un au moins, et de préférence deux au moins, sont réalisés comme défini plus haut.

Le dispositif peut comporter au moins trois chambres microfluidiques reliées en série par au moins deux systèmes d'interconnexion, dans lequel l'épaisseur desdits systèmes d'interconnexion est inférieure à l'épaisseur desdites trois chambres microfluidiques, et au moins l'une desdites chambres microfluidiques présente une épaisseur comprise entre l'épaisseur d'au moins une des autres chambres microfluidiques et celle desdits systèmes d'interconnexion.

Le dispositif peut comporter au moins trois chambres microfluidiques reliées en série par au moins deux système d'interconnexion d'orientation différentes, notamment perpendiculaires.

L'invention a encore pour objet, selon un autre de ses aspects, un procédé de culture cellulaire, notamment de cellules neuronales, dans lequel on ensemence avec des cellules neuronales au moins une chambre microfluidique (2a, 2b) d'un dispositif tel que défini plus haut, lesdites cellules pouvant, lors de leur multiplication et/ou de leur différenciation, donner lieu à des extensions cellulaires.

On peut ensemencer au moins deux chambres microfluidiques avec des cellules neuronales et les axones des cellules de l'une des chambres éprouvent moins de difficultés à se développer vers l'autre chambre que ceux des cellules de l'autre chambre en raison de la forme du système d'interconnexion.

On peut ensemencer au moins une chambre microfluidique avec une culture cellulaire présentant au moins deux types cellulaires, et les axones des cellules d'au moins l'un desdits types cellulaires éprouvent moins de difficultés à se développer au sein du système d'interconnection que les axones des cellules d'au moins un second type cellulaire.

On peut réaliser une axotomie grâce à un canal traversant le système d'interconnexion, comme indiqué plus haut. Les cellules contenues dans l'une des chambres peuvent être mises en présence d'un composé. Le canal précité peut être rempli d'un liquide constituant un obstacle vis-à-vis de la progression du composé vers l'autre chambre par l'intermédiaire du système d'interconnexion.

Un dispositif selon l'invention peut permettre de compartimenter des neurones isolés ou de reconstruire des réseaux orientés de neurones. Les architectures des dispositifs permettent d'appliquer sélectivement des fluides de composition variée, spécifiquement sur chacun des sous compartiments des réseaux de neurones (par exemple si l'on considère un neurone de type A connecté à un neurone de type B ; compartiment somato-dendritique du neurone A, portion d'axone du neurone A, compartiment somato-dendritique du neurone B, axone du neurone B ...).

Il est ainsi possible de modéliser des conditions dégénératives en appliquant un stress sur une quelconque partie du réseau, et d'en suivre les conséquences. Ce stress peut comporter par exemple l'axotomie des axones du neurone A, l'application de molécules toxiques sur le compartiment somato-dendritique ou axonal médian du neurone A, sur le compartiment somato-dendritique du neurone B ...). Le stress peut comporter l'application de molécules toxiques (de nature organique ou inorganique), neurotoxiques, synaptotoxiques, axotoxiques .., de protéines normales ou mutées ou chimères ajoutées dans le milieu cellulaire ou transférées génétiquement, de virus ou agents infectieux neurotropes (ou non), de modifications des conditions physicochimiques, ou physiques (pression, température, ondes ..).

L'application de ces stress pourra provoquer une dégénérescence progressive de tout ou partie des composants cellulaires (modification de la transmission synaptique, rétraction synaptique, dégénérescence axonale, modification des paramètres de transport axonal, dégénérescence somato-dentritique...) dont les conséquences pourront être suivies par l'analyse de bio marqueurs ou de la morphologie cellulaire.

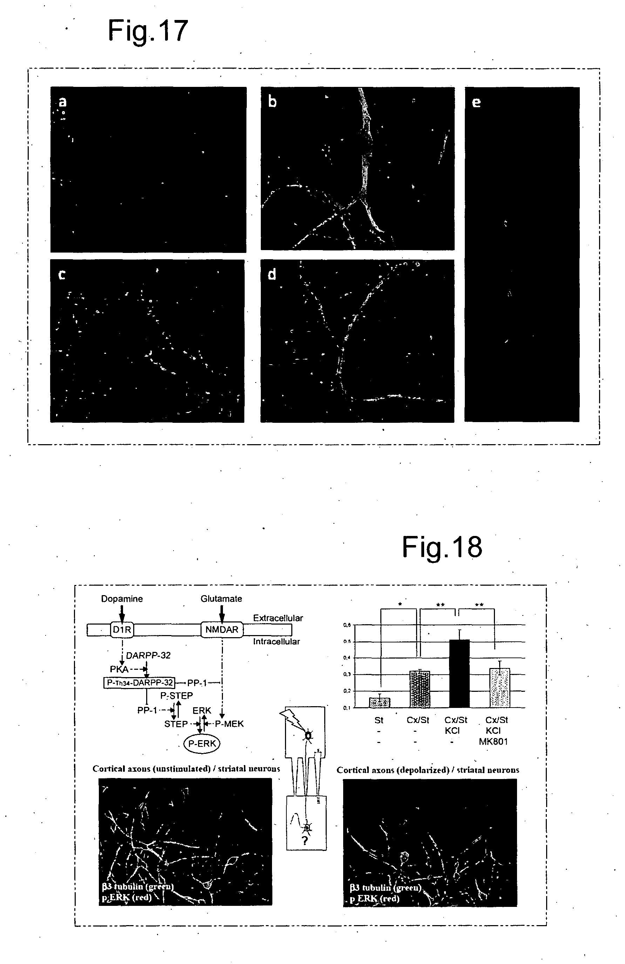

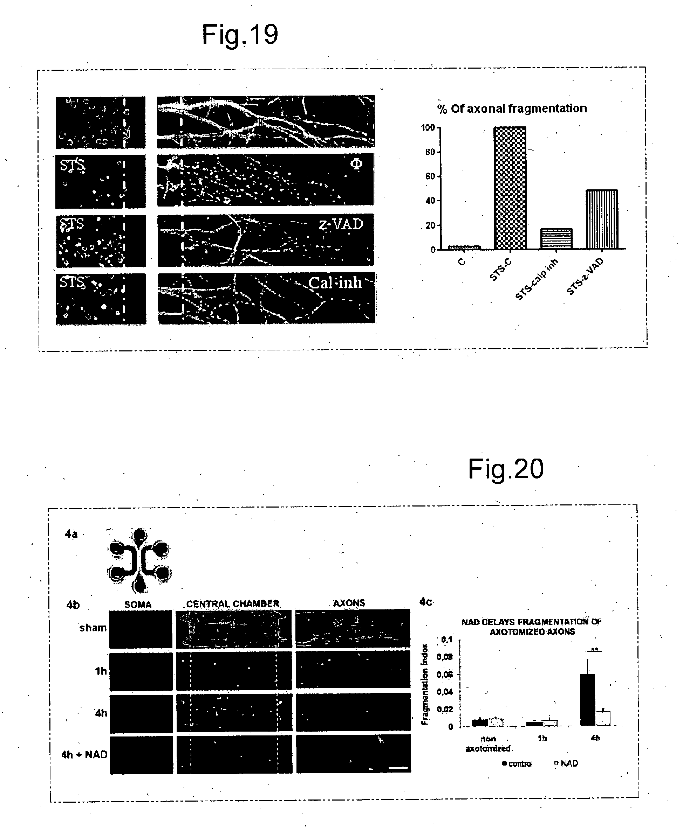

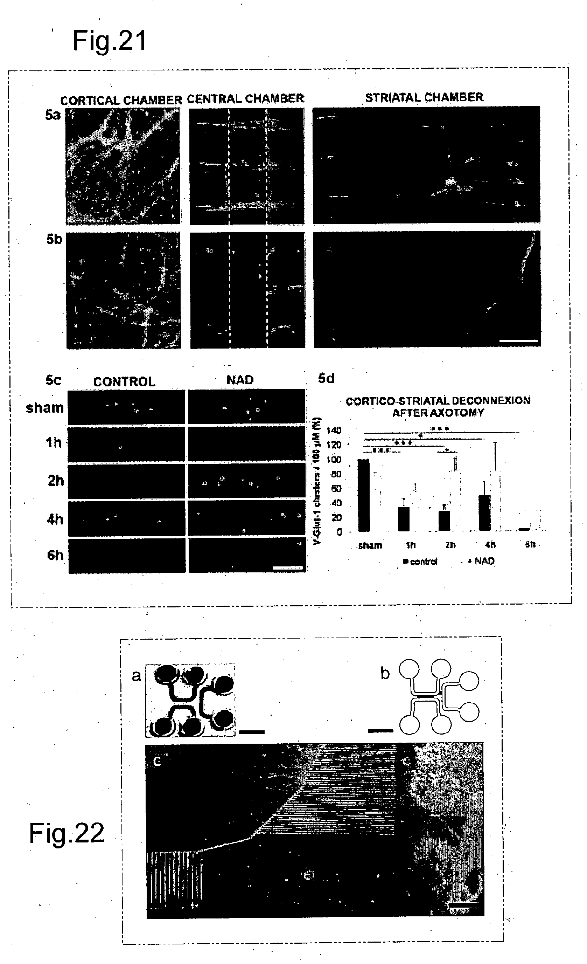

La survenue de ces processus dégénératifs pourra être bloquée ou freinée en appliquant localement sur une composante du réseau des molécules dont on souhaite évaluer les propriétés pharmacologiques (molécules organiques issues de la synthèse chimique ou extraction naturelle, protéines (facteurs neurotrophiques ..), polymères naturels ou non. Leurs propriétés pourront être évaluées sur la capacité à restaurer i) la fonctionnalité synaptique (par exemple illustrée à la Figure 18 par les expériences de mobilisation, dans les neurones striataux, de la kinase erk, après stimulation corticale) ii) la dégénérescence synaptique (par exemple illustrée à la Figure 21 par les expériences de suivi des structures pré-synaptiques VGlut-1 dans les expériences d'axotomie), iii) la dégénérescence axonale ou la dégénérescence somatique (par exemple illustrée aux Figures 19 et 20 par les expériences d'axotomie de neurones corticaux et d'application de staurosporine somatique). De la même façon il est possible d'évaluer le potentiel de molécules visant à régénérer les structures détruites (facteurs neurotrophiques ...)

Un dispositif selon l'invention peut convenir tout particulièrement pour étudier la dégénérescence axonale ou synaptique et effectuer un criblage de substances actives sur cette dégénérescence

Le dispositif peut être utilisé pour une compartimentalisation neuronale efficace, l'imposition d'une directionalité à la croissance axonale, la sélection entre différents types d'axones, et/ou la création de réseaux de cellules impliquant différents types de cellules contenus dans des chambres différentes, et présentant des connexions dirigées par l'intermédiaire des extensions cellulaires.

Le dispositif peut être utilisé pour imposer de façon dynamique certains stimuli physiques ou chimiques à un ou plusieurs sous-compartiments cellulaires sélectionnés, le biomarquage spécifique de certains de ces sous-compartiments et l'analyse du contenu biologique de certains de ces sous-compartiments.

Selon un aspect, le dispositif microfluidique comprend au moins deux chambres microfluidiques de culture cellulaire, dont la dimension est compatible avec le développement de soma cellulaires, séparées par une zone d'interconnexion microfabriquée dont la géométrie peut affecter la direction, la cinétique, le nombre, le type, les interconnexions, de fibres axonales ou plus généralement d'extensions cellulaires. Différents types de microstructures peuvent être utilisées à cet effet, et divers exemples sont proposés ci-dessous.

Différents types de microstructures du système d'interconnexion fludique peuvent avoir différents types d'effets ou exercer différents types de sélection sur la croissance des extensions cellulaires, et peuvent être sélectionnés et éventuellement combinés en fonction des effets souhaités.

Des structures comportant des canaux présentant un rétrécissement asymétrique vont favoriser la croissance d'extensions cellulaires dans un sens, en général celui qui va du plus large au plus étroit.

Des structures comportant des microcanaux présentant une tortuosité permettront de retarder, accélérer ou sélectionner certains types particuliers d'axones appartenant à des sous-types neuronaux. L'invention a ainsi pour objet l'utilisation d'un dispositif comportant une telle structure dans ce but.

Des structures à base d'obstacles, par exemple de micro-piliers, peuvent permettre une compartimentalisation axonale ou dentritique, tout en permettant de réguler la formation de connexions inter-axonales. L'invention a ainsi pour objet l'utilisation d'un dispositif comportant une telle structure dans ce but.

On propose des dispositifs de culture microfluidiques, comportant en sus des systèmes d'interconnexion ci-dessus, des sous-chambres de culture de cellules individuelles permettant de contrôler précisément le positionnement des somas cellulaires. Optionnellement, ces sous-chambres présentent une surface traitée de façon à favoriser l'adhésion d'au moins un certain type de soma cellulaire.

Le dispositif peut être utilisé avec tout type de cellules pouvant, lors de leur multiplication et/ou de leur différenciation, donner lieu à des extensions cellulaires. Concernées sont en particulier des cellules souches, des cellules neuronales, des cellules souches neuronales, ainsi que toutes les cellules pouvant donner naissance, lors de leur différenciation, à des cellules neuronales et à des sous-types neuronaux spécialisés.

Le dispositif peut être utilisé pour la reconstruction orientée de réseaux neuronaux du plus simple au plus complexe, impliquant éventuellement différents sous-types neuronaux, et/ou une combinaison de neurones et de cellules non-neuronales, et/ou différents types d'interactions axonales et/ou dendritiques entre neurones.

Le dispositif peut être utilisé pour reconstruire et d'étudier les interactions entre cellules neuronales et cellules non-neuronales.

Le dispositif peut être utilisé pour adresser des compartiments neuronaux spécifiques avec des réactifs et/ou des médicaments, de collecter pour analyse des biomarqueurs provenant de certains de ces compartiments.

Le caractère transparent des chambres et des systèmes d'interconnexion peut faciliter l'analyse in situ, optionnellement en temps réel, desdits compartiments cellulaires tels que axones, dendrites, synapses, soma, avec des outils de biologie moléculaire ou cellulaire tels que, à titre d'exemples non-limitatifs, l'immunohistochimie, l'hybridation d'acides nucléiques, l'amplification d'acides nucléiques, l'électrochimie, l'électrophysiologie. Entre dans le cadre de l'invention la réalisation de telles études pendant le développement neuronal, dans des états neuronaux ou patholologiques.

Le dispositif est particulièrement bien adaptée aux études comparatives, le criblage à haut contenu (high content screening), le criblage à haut débit (high throughput screening), la recherche de médicaments ou les études toxicologiques.

Il est également particulièrement bien adapté à l'étude de l'effet de neurotoxiques naturels ou artificiels, de signaux apoptotiques, de neuroprotecteurs naturels ou artificiels.

D'une manière générale, le dispositif se prête bien à l'étude détaillée de la présence, dans des compartiments cellulaires spécifiques, de biomarqueurs.

Par "biomarqueur" on entend tout type d'information relative à l'état biologique d'une cellule. Des exemples typiques de biomarqueurs couramment utilisés en biologie, en biologie cellulaire et en médecine, sont la présence d'une protéine, sa concentration, son taux d'expression, la présence ou la concentration d'un métabolite, d'un neurotransmetteur, d'une séquence nucléotidique, d'une enzyme où encore la localisation d'une protéine, sa distribution spatiale dans le compartiment cellulaire, ou toute combinaison de ces critères.

Par « déterminer » on entend se référer à une détection quantitative ou qualitative.

Par « valeur contrôle », « intervalle de valeurs contrôles » ou « détermination contrôle », on entend se référer à une valeur, un intervalle de valeurs ou une détermination d'un paramètre déterminé dans des conditions dites de base ou normale. Habituellement, une telle condition est obtenue en l'absence de l'élément dont l'effet est à déterminer.

Par exemple, en ce qui concerne le degré d'expression d'un biomarqueur, la valeur contrôle, l'intervalle de valeurs contrôles, ou la détermination contrôle, peuvent être une valeur, un intervalle de valeur ou une détermination correspondant au degré d'expression dudit biomarqueur dans des cellules en l'absence de stimulus induisant la dégénérescence cellulaire.

Ces biomarqueurs peuvent être détectés par diverses méthodes, optiques, en particulier l'imagerie de fluorescence, ou électrique, en particulier l'électrophysiologie.

De nombreuses méthodes sont connues de l'homme de l'art, et en particulier des biologistes cellulaires et des neurologues, pour identifier des biomarqueurs dans des cellules, En fait, un des avantages majeurs de l'invention, est de permettre l'utilisation in vivo de la plus large panoplie de méthodes de détection et d'analyse de biomarqueurs connue en biologie cellulaire et de pouvoir pour la première fois le faire sur des neurones disposés dans des architectures précédemment inaccessibles.

L'invention pourra être mieux comprise à la lecture de la description détaillée qui va suivre, d'exemples de mise en œuvre non limitatifs de celle-ci, et à l'examen du dessin

### Figures et discussion détaillée de l'invention

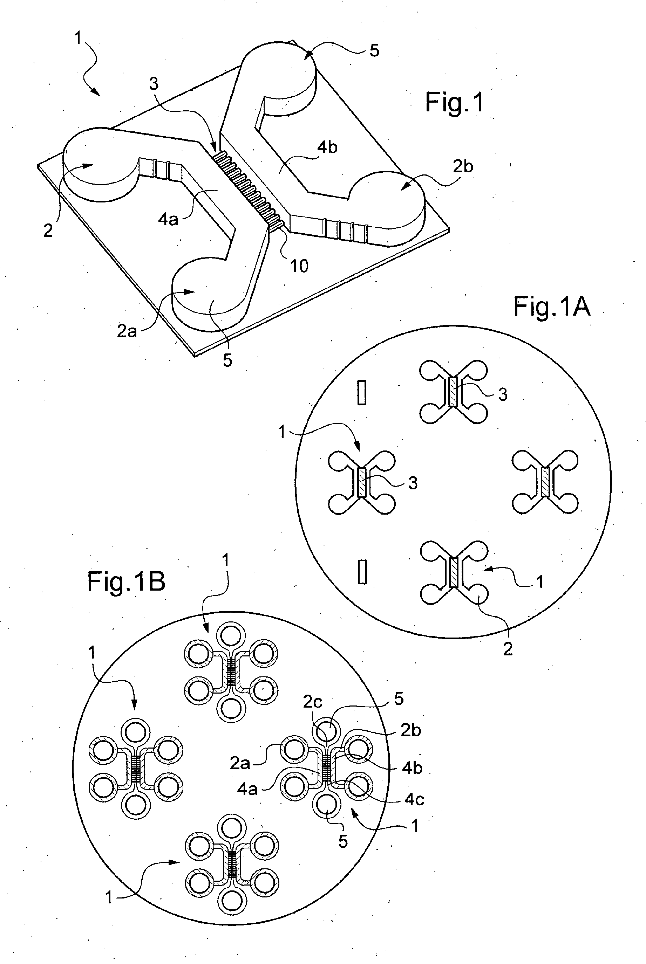

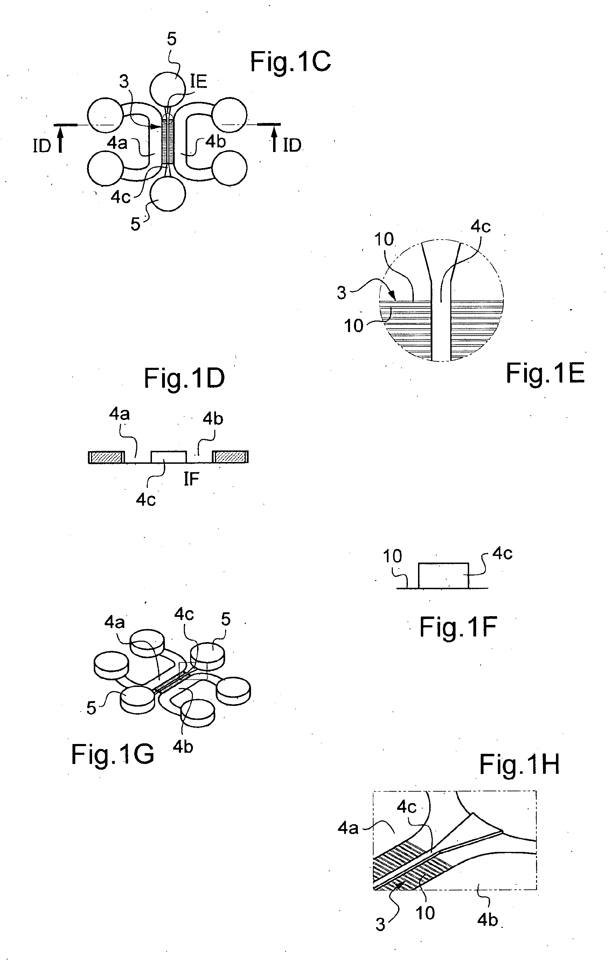

On a représenté à la figure 1, de manière schématique, un exemple de dispositif microfluidique 1 réalisé conformément à l'invention, encore appelé "puce".

Ce système microfluidique 1 comporte dans l'exemple considéré des chambres microfluidiques 2, en l'espèce au nombre de deux et référencées 2a et 2b, destinées à être ensemencées par des première et deuxième cultures cellulaires respectivement.

Le système microfluidique 1 comporte également un système d'interconnexion fluidique 3 reliant les première et deuxième chambres 2a et 2b et permettant à des extensions cellulaires de s'étendre d'une chambre vers l'autre chambre.

Les chambres microfluidiques 2 sont par exemple réalisées avec deux portions parallèles respectives 4a et 4b entre lesquelles s'étend le système d'interconnexion fluidique 3.

Les portions 4a et 4b, encore appelées macrocanaux, sont par exemple, comme illustré, reliées à leurs extrémités à des portions 5 plus larges (encore appelées réservoirs), par exemple de forme cylindrique.

Le dispositif 1 peut être réalisé sur tout support adapté, monomatière ou multimatériau. Ce support peut comporter, comme illustré à la figure 1A, plusieurs dispositifs de culture cellulaire 1 selon l'invention.

Dans l'exemple de la figure 1A, les dispositifs 1 sont réalisés sur un substrat basal qui est par exemple transparent, étant par exemple une lamelle de microscope circulaire, par exemple de 42 mm de diamètre. L'invention n'est pas limitée à un matériau et à une forme particulière.

Le matériau dans lequel les chambres sont formées est par exemple du PDMS, collé sur la plaque de verre constituée par la lamelle de microscope par exemple, ou d'un autre matériau qui définit le fond des chambres.

Pour remplir les chambres, on peut par exemple percer le matériau dans lequel les chambres sont formées, au niveau des portions plus larges 5. On forme par exemple des trous de 4 mm dans celles-ci.

On peut introduire un liquide dans les chambres à l'aide d'une pipette ou d'un perfuseur. On peut introduire par exemple entre 10 et 100 µL de liquide dans chaque chambre 2a et 2b.

Lors de l'utilisation du dispositif, il peut n'exister aucun écoulement permanent de liquide entre les chambres 2a et 2b.

Sur la figure 1, les portions 5 sont cylindriques, mais d'autres formes sont possibles.

Les dispositifs 1 peuvent être autonomes du point de vue fluidique (fluidiquement autonomes), c'est-à-dire qu'une fois les différents milieux introduits dans les chambres, il n'existe pas de communication fluidique du dispositif avec l'extérieur.

Sur la figure 1B, les dispositifs 1 comportent trois chambres 2a, 2b et 2c par dispositif 1 microfluidique.

La troisième chambre 2c (encore appelée chambre centrale, auquel cas les chambres 2a et 2b peuvent être appelées chambres distales) comporte une portion 4c rectiligne, encore appelée canal, qui s'étend entre les portions 4a et 4b des chambres 2a et 2b. Cette portion 4c relie deux portions plus larges 5 de la chambre 2c.

Les portions 4a, 4b, 4c ont par exemple une largeur d'au moins 55 µm.

Sur les figures 1A et 1B, on n'a pas représenté dans un souci de clarté du dessin le système d'interconnexion fluidique 3.

La portion 4c peut être remplie d'un liquide qui ne gêne pas la progression des extensions cellulaires mais peut limiter la diffusion d'un composé introduit dans l'une des chambres vers l'autre chambre.

La portion 4c peut encore être utilisée pour effectuer une axotomie, en la remplissant d'un détergent par exemple.

La portion 4c peut s'étendre selon un axe longitudinal contenu dans un plan de symétrie pour les première et deuxième chambres, mais d'autres configurations sont possibles.

Le système d'interconnexion fluidique 3 peut être réalisé de diverses façons, dans le but de privilégier la progression d'au moins un premier type d'extension cellulaire par rapport à au moins un deuxième type d'extension cellulaire, les premier et second types d'extension cellulaire différant par exemple par la chambre microfluidique dont ils proviennent ou par le type cellulaire dont ils sont l'extension. Les progressions peuvent avoir lieu par exemple sans courant de fluide dans le système d'interconnexion.

D'une manière générale, le système d'interconnexion fluidique peut comporter une pluralité de microcanaux juxtaposés, qui sont reliés à leurs extrémités aux chambres cellulaires, soit directement, soit par l'intermédiaire de chambres monocellulaires comme cela sera précisé plus loin.

Le système d'interconnexion fluidique peut comporter par exemple entre 1 et 1000 microcanaux, lesquels peuvent avoir diverses formes.

Tous les microcanaux d'un même système d'interconnexion fluidique peuvent avoir la même forme, chacun étant par exemple la réplique d'un autre par exemple par une translation ou une rotation ou une transformation géométrique plus complexe.

Le système d'interconnexion fluidique peut comporter une pluralité de microcanaux dont les axes longitudinaux sont parallèles et par exemple perpendiculaires aux macrocanaux 4a et 4b, voire 4c, des chambres cellulaires 2 qu'ils relient.

Le système d'interconnexion fluidique peut comporter des microcanaux ayant une forme asymétrique, notamment lorsqu'observé en vue de dessus.

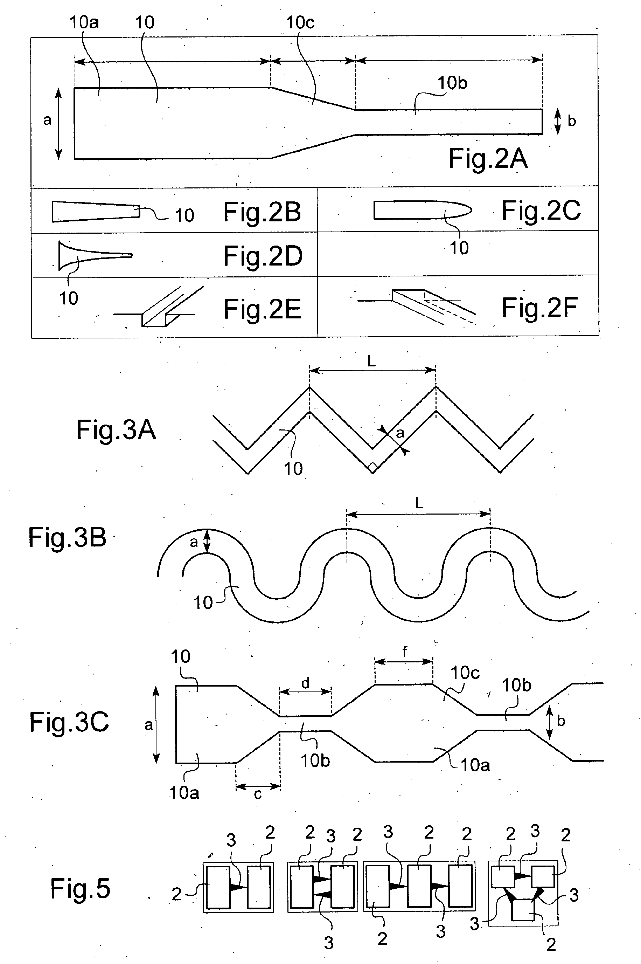

Sur la figure 2A, on a représenté un premier exemple de microcanal 10 du système d'interconnexion fluidique 3, présentant une forme asymétrique, avec une portion plus large 10a de largeur a et une portion plus étroite 10b de largeur b, reliées par une portion intermédiaire 10c, ayant par exemple une largeur qui décroît linéairement.

On a remarqué de façon étonnante qu'en utilisant des dispositifs présentant des canaux asymétriques de longueurs différentes, on était capable de sélectionner les axones non seulement en fonction de la chambre de laquelle ils proviennent, mais également en fonction de leur sous-type cellulaire.

Par exemple, en utilisant des canaux d'au moins 1200 µm de long, les neurones de granules cérébraux ne peuvent envoyer d'axones dans la chambre distale, tandis que les neurones de projection le peuvent.

Egalement, on a observé que des axones ne pénètrent pas ou pénètrent difficilement dans un canal dont la largeur est inférieure ou égale à 2 µm, ce qui permet d'effectuer des interconnexions fluidiques imperméables aux axones.

Les figures 2B à 2D représentent également d'autres formes possibles pour les microcanaux 10, par exemple une forme trapézoïdale en vue de dessus comme illustré sur la figure 2B, une forme en ogive comme illustré à la figure 2C ou une forme en pavillon comme illustré à la figure 2D. La largeur du microcanal 10 peut varier de façon linéaire ou non, lorsque l'on se déplace le long de l'axe longitudinal de celui-ci.

Les microcanaux 10 peuvent être formés par des rainures en partie basse du dispositif, comme illustré à la figure 2E, ou par des rainures formées en partie haute, comme illustré sur la figure 2F.

Les microcanaux 10 ont par exemple une longueur comprise entre 10 µm et 10 000 µm, de préférence entre 50 µm et 2000 µm, par exemple de l'ordre de 500 µm.

Les microcanaux 10 ont par exemple une épaisseur comprise entre 1 et 5 µm, par exemple de l'ordre de 3 µm.

La largeur des microcanaux 10, dans leur partie la plus large, est par exemple comprise entre 5 et 100 µm et dans la partie la moins large est comprise entre 1 et 10 µm. La largeur des microcanaux, dans la partie la moins large, est par exemple au plus égale à la moitié de leur largeur dans la partie la plus large.

Dans des exemples de réalisation, la largeur dans la partie la plus large vaut 10 µm et celle dans la partie la moins large 3µm. Avec de telles valeurs, on peut observer de façon surprenante et spectaculaire une sélectivité avoisinant 1000, c'est-à-dire que les cellules contenues dans la chambre cellulaire en contact avec la partie la plus large du microcanal envoient au moins 1000 fois plus d'axones que celles contenues dans la chambre cellulaire en contact avec la partie étroite du microcanal.

Les microcanaux du système d'interconnexion fluidique peuvent avoir chacun une forme rectiligne ou en variante une forme non rectiligne.

Sur la figure 3A, on a représenté un microcanal 10 ayant une forme en zigzag, avec par exemple des portions consécutives disposées à angle droit les unes par rapport aux autres. La période L du motif en zigzag est par exemple comprise entre 5 µm et 100 µm, et de préférence entre 10 et 50 µm

Sur la figure 3B, on a représenté un microcanal 10 ayant une forme ondulée, composée par exemple d'une succession de portions hémicirculaires. Suivant le type neuronal à sélectionner, la période L du motif ondulé est par exemple comprise entre 3 et 5 µm, ou entre 5 et 10 µm, ou encore entre 10 et 30 µm.

Ce type de canaux peut être utile pour sélectionner des sous-types d'axones. Par exemple, en utilisant des canaux avec de faibles rayons de courbure, on a observé que les axones corticaux sont arrêtés, alors que les axones de l'hippocampe ou les axones striataux sont capables de traverser ces canaux.

On a représenté à la figure 3C un exemple de microcanal 10 qui comporte une succession de portions élargies 10a et de portions rétrécies 10b. Les portions rétrécies 10a et 10b sont par exemple de largeur constante tandis que les portions élargies présentent des entrées et sorties respectivement divergentes et convergentes et une portion médiane 10c de largeur constante a, par exemple comprise entre 5 µm et 50 µm. La largeur b des portions rétrécies 10b est par exemple comprise entre 2µm et 10 µm

La longueur d des portions rétrécies 10b est par exemple comprise entre 10 et 500 µm. La longueur c des entrées et sorties est par exemple comprise entre 10 et 500 µm. La longueur f de la portion 10c est par exemple comprise entre 10 et 500 µm.

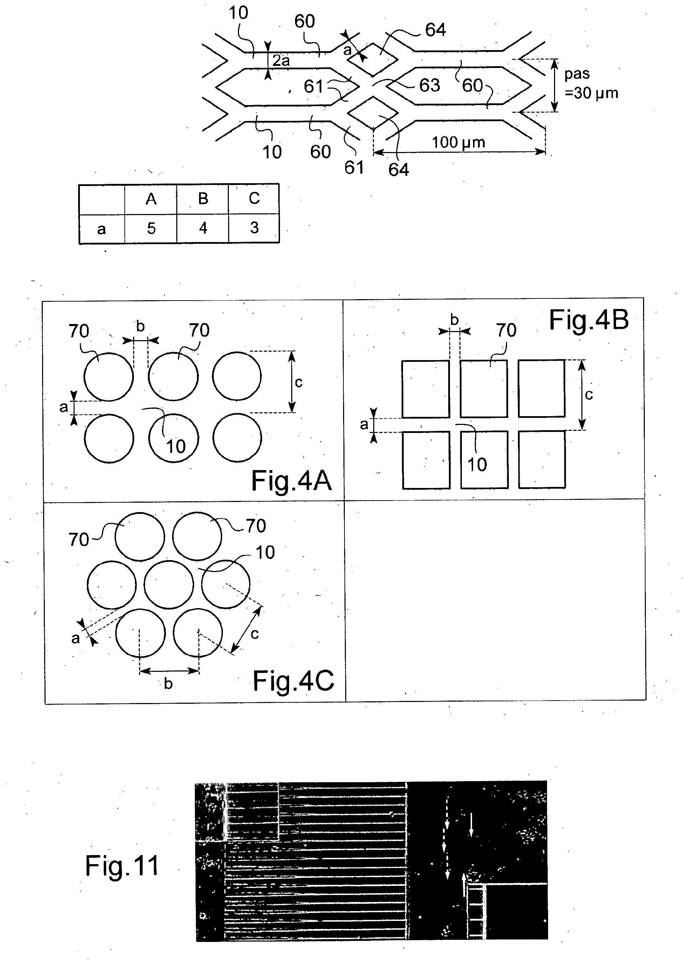

Les figures 4A à 4C donnent des exemples de système d'interconnexion 3 du type constitué de réseaux d'obstacles.

Sur ces figures, on voit que l'on peut réaliser des obstacles 70, par exemple sous la forme d'îlots circulaires (encore appelés micro-piliers) comme illustré sur la figure 4A ou de pavés de forme polygonale, par exemple rectangulaire comme illustré sur la figure 4B.

Les obstacles 70 peuvent avoir différentes dispositions, par exemple être agencés sous forme de réseau avec positionnements réguliers dans les deux dimensions du plan comme sur la figure 4A ou 4B. Les obstacles 70 peuvent encore être sous la forme d'agencements où les centres des îlots sont disposés comme les sommets de polygones, comme illustré à la figure 4C.

La disposition de la figure 4A correspond à un mode de réalisation privilégié, dans lequel il n'est pas possible de faire circuler entre les obstacles 70 une ligne ne présentant pas de rayon de courbure inférieur au rayon des obstacles. Une telle disposition peut être utilisée pour séparer des axones corticaux et striataux. Les valeurs a, b et c valent par exemple entre 2 et 10 µm, entre 2 et 10 µm, et entre 5 et 100 µm, respectivement, et préférentiellement entre 2 et 5 µm, entre 2 et 5 µm, et entre 5 et 50 µm.

La figure 5 donne des exemples, non-exhaustifs, de dispositions utilisables pour former à l'aide de l'invention des architectures neuronales complexes et orientées. Les petits rectangles représentent, de façon schématique, des chambres de culture cellulaire 2. Les flèches représentent, également de façon schématique, les systèmes d'interconnexion et leur directionalité. Ces systèmes dirigés peuvent être utiles par exemple pour étudier la croissance axonale, la synaptogénèse, le transport rétrograde et antérétrograde, ou la signalisation cellulaire, pour étudier les processus neurodégénératifs, comme les maladies d'Alzheimer, de Huntington, de Parkinson, en particulier celles dans lesquelles des sous-types spécifiques de neurones montrent des signes de dysfonctionnement synaptiques précoce.

Les systèmes d'interconnexion fluidique peuvent être disposés dans l'alignement l'un de l'autre ou en variante peuvent être orientés dans des directions différentes.

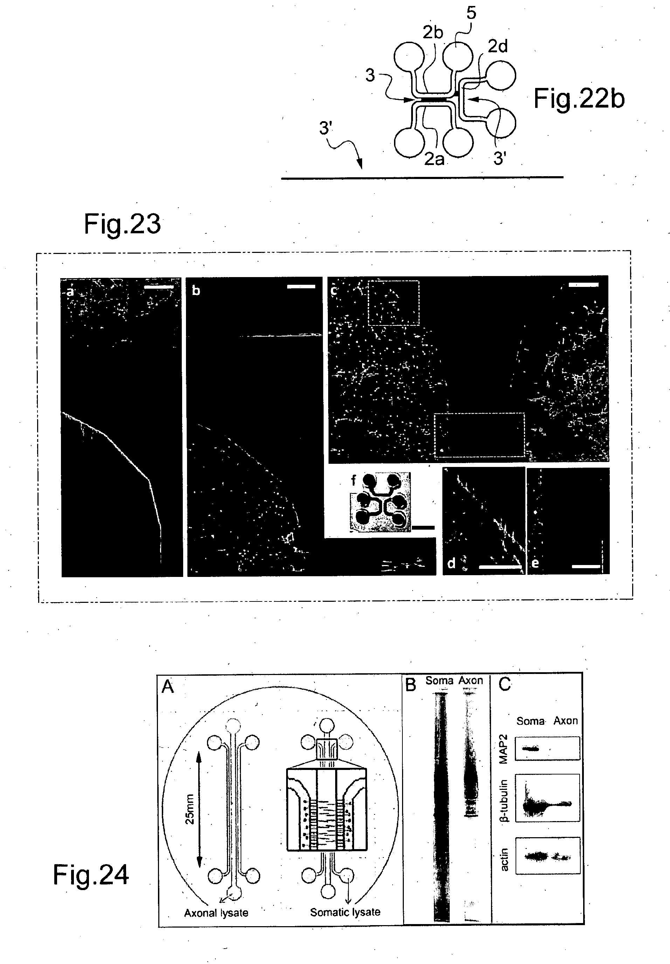

La figure 22 représente en b un exemple à trois chambres 2a, 2b et 2d, les chambres 2a et 2b étant reliées par un premier système d'interconnexion fluidique 3 et les chambres 2b et 2d par un second système d'interconnexion fluidique 3', que l'on voit également sur l'image c. Les microcanaux des différents systèmes d'interconnexion sont perpendiculaires entre eux. L'écartement entre les microcanaux du système d'interconnexion 3 peut être différent de celui entre les microcanaux du système d'interconnexion 3'.

Les microcanaux du système d'interconnexion fluidique se raccordent par exemple au coude qui relie le macrocanal de la chambre 2b à la portion plus large 5 adjacente.

Si l'on souhaite pouvoir infuser des réactifs auprès de certains compartiments neuronaux, ou collecter des produits, sans perturber l'organisation neuronale, selon l'une des variantes de l'invention, le système d'interconnexion est délimité sur au moins une partie de sa surface par une paroi poreuse permettant l'échange avec un compartiment annexe de molécules et d'ions, mais ne permettant pas l'échange avec ledit compartiment annexe de cellules. Cette paroi peut être constituée par exemple en intercalant une membrane entre deux couches de matériau constituant, respectivement, la partie basse et la partie haute du canal, et en réservant dans la partie haute un canal annexe, en contact avec ladite membrane, et capable d'amener ou de collecter des produits.

Cette membrane peut être constituée in situ, par exemple par photopolymérisation.

Enfin, elle peut aussi être constituée par un réseau d'obstacles très rapprochés, par exemple présentant une distance inférieure à 2 µm, qui ne laisse pas passer les axones.

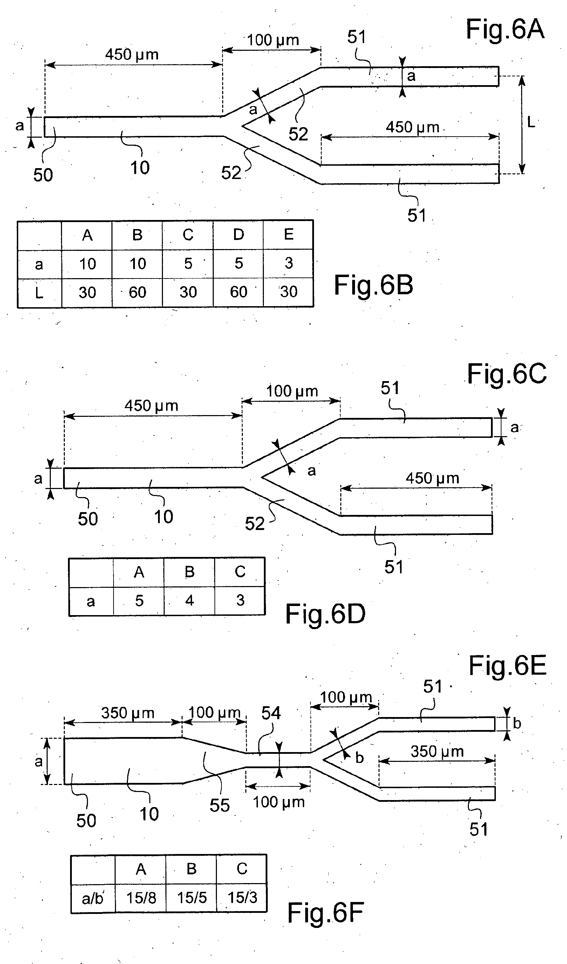

A la figure 6A, on a représenté un exemple de microcanal 10 avec bifurcation qui comporte une première portion 50 qui se raccorde à deux branches 51. La portion 50 peut présenter une largeur *a* de même que les branches 51. Les motifs avec bifurcations permettent par exemple la connexion ou la division axonale. Les axes longitudinaux des branches 51 sont par exemple écartés, à l'extrémité opposée à l'extrémité d'entrée de la portion 50, d'une distance L. L'axe longitudinal de la portion 50 est par exemple contenu dans un plan médian pour les branches 51. Ces dernières peuvent comporter deux portions 52 qui divergent, par exemple sur une longueur de 100 µm.

A la figure 6B, on a représenté un tableau de valeurs pour les paramètres a et L pour différents exemples de configurations A à E.

A la figure 6C, le microcanal 10 est réalisé avec une portion 50 dont la largeur 2a est par exemple double de celle des branches 51. Le tableau de la figure 6D donne quelques exemples de valeurs pour la largeur a, dans différentes configurations A à C.

La figure 6E représente un microcanal 10 comportant une portion 50 qui se rétrécit avant de se scinder en deux branches 51. La portion 50 passe par exemple d'une largeur *a* à une largeur *b* et les branches 51 ont par exemple la même largeur que celle de la portion rétrécie 54.

La longueur de la portion convergente 55, par laquelle la portion 50 se raccorde à la portion rétrécie 54, vaut par exemple 100 µm.

Le tableau de la figure 6F donne différents exemples du ratio a/b pour différentes configuration A à C. La présence de la portion rétrécie 54 permet de focaliser le faisceau.

La configuration de la figure 6C permet de conserver le débit. La contrainte sur le filopode est à priori plus grande que dans l'exemple de la figure 6A, ce qui permet d'espérer sa fission.

La figure 6G présente une configuration ayant des propriétés similaires à celles de la figure 6C en ce qui concerne les contraintes mécaniques sur la zone en Y. La configuration de la figure 6G permet aussi de tester le comportement de faisceaux d'axones qui se croisent.

Dans la configuration de la figure 6G, les microcanaux 10 comportent des portions rectilignes parallèles 60 qui se raccordent à des branches 61 qui divergent puis convergent pour se raccorder à une nouvelle portion rectiligne 60.

Deux branches 61 de deux canaux adjacents se rejoignent en 63, les branches ménageant entre elles un îlot 64. L'écartement entre les axes longitudinaux des portions rectilignes 60 est par exemple de 30 µm et le pas dans la direction longitudinale est par exemple de 100 µm. La largeur d'une portion 60 est par exemple double, égale à 2a de celle d'une branche 61.

Le tableau de la figure 6H donne différents exemples de valeurs de la largeur *a* dans des configurations A à C.

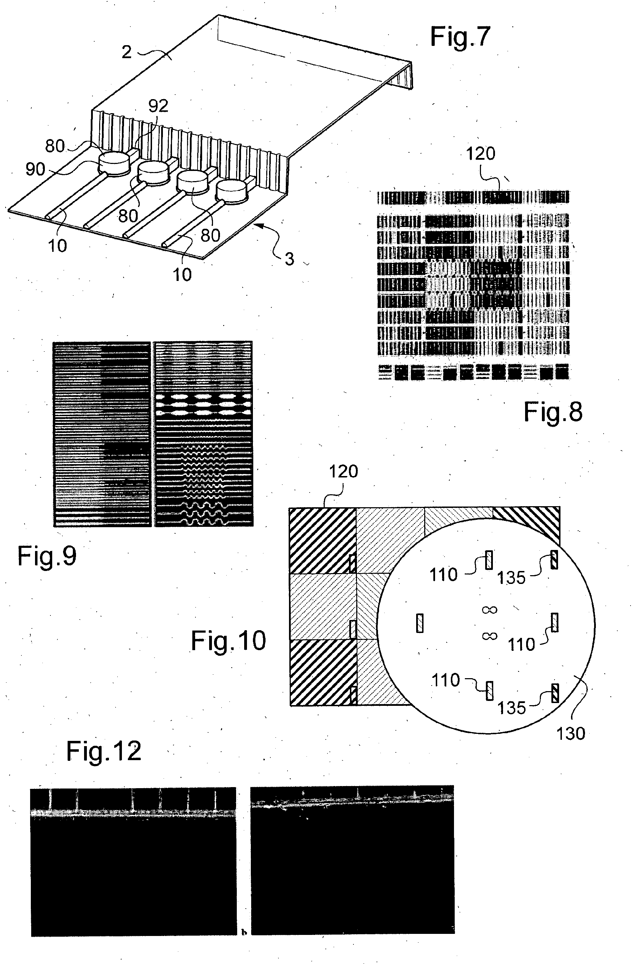

Sur la figure 7, on a illustré la possibilité de réaliser des chambres monocellulaires 80 entre les chambres 2. Les chambres monocellulaires 80 communiquent par exemple avec une extrémité 90 d'un microcanal associé 10 et du côté opposé avec un canal de liaison 92 dont la section est plus grande que celle du microcanal 10 qui se raccorde directement à la chambre 2. La chambre 2 fait par exemple 55 µm de hauteur, les chambres monocellulaires 12 µm et les microcanaux 10, 30 µm.

Les chambres monocellulaires 80 peuvent piéger les somas des neurones devant l'entrée des micro-canaux. Pour réaliser ces structures, une troisième couche de résine peut s'avérer nécessaire. Leur taille caractéristique pourrait varier de 10 à 100 µm pour accueillir un ou plusieurs somas.

En ce qui concerne la fabrication des dispositifs selon l'invention, de nombreuses méthodes de microlithographie peuvent être utilisées, par exemple celles décrites par Patrick Tabeling. Introduction à la microfluidique Belin.

Selon un mode de réalisation privilégié, le dispositif est fabriqué grâce aux méthodes de lithographie douce, comme décrit dans Whitesides, G. M. ; Ostuni, E. ; Takayama, S. ; Jiang, X. ; Ingber, D. Soft lithography in biology and biochemistry., Annu. Rev. Biomed. Eng. 2001, 3, 335-373. On peut utiliser un élastomère, le PDMS (polydiméthylsiloxane) qui, une fois réticulé, possède des propriétés bien adaptées à la biologie cellulaire et moléculaire : transparent, peu réactif, biocompatible.

Le PDMS n'est pas une surface sur laquelle les neurones adhèrent naturellement. Quel que soit l'environnement de culture (boîte de petri ou puce microfluidique), il peut s'avérer utile de traiter chimiquement la surface. Le moyen le plus couramment utilisé consiste à utiliser de la poly-lysine, qui s'adsorbe aux parois et permet l'adhésion cellulaire. Il est aussi possible d'utiliser d'autres protéines d'adhésion comme la fibronectine ou le collagène, mais ces substances ne s'adsorbent pas suffisamment au PDMS de façon naturelle pour permettre l'adhésion cellulaire, contrairement à la poly-lysine. Il peut être nécessaire de les lier chimiquement au PDMS. On peut donc utiliser un cross-linker 2 photo-activable : lorsque celui-ci reçoit un rayonnement UV, l'énergie reçue lui permet de créer une liaison avec le PDMS. De nombreux réticulants ou crosslinkers, peuvent être utilisés dans l'invention, tels que, à titre d'exemples non limitatifs :
- Sulfo-sanpah : Le sulfo-sanpah (N-Sulfosuccinimidyl-6-[4'-azido-2'-nitrophenylamino] hexanoate) est une molécule qui possède à l'une de ses extrémités un groupement nitrophenyle azide photo-activable, et à l'autre extrémité un groupement ester N-sulfosuccinimidyle qui a pour particularité d'être très réactif avec les fonctions amines (amidation), très courantes dans les protéines.
- Benzophénone et BBTA : La benzophénone est un initiateur radicalaire qui s'adsorbe fortement sur le PDMS. Lorsqu'il est photo-activé, il peut créer une liaison avec une molécule contenant des hydrogènes et ainsi la greffer sur la surface. La BBTA ((4-benzoylbenzyl)triméthylammonium) est une version hydrophile de la benzophénone, mais son fonctionnement est *a priori* le même, à ceci près qu'il peut être mis dans la même solution que les protéines puisqu'il est hydrophile. La réaction a lieu alors que les deux molécules sont dans l'eau, contrairement à la benzophénone qui doit d'abord être adsorbée sur le PDMS.

Selon un mode de réalisation privilégié, utile en particulier en conjonction avec les applications dans lesquelles on souhaite favoriser l'adhésion de cellules ou de compartiments cellulaires spécifiques à des endroits spéciriques, on peut utiliser une photo-activation localisée.

On peut par exemple utiliser un masque pour n'éclairer aux UV qu'aux endroits désirés, afin de créer des motifs de protéines d'adhésion comme décrit dans Jenny Fink, Manuel Théry, Ammar Azioune, Raphael Dupont, François Chatelain, Michel Bornensa and Matthieu Piel. Comparative study and improvement of current cell micro-patterning techniques. Lab on a Chip, 2007, 7,672-680.

Selon un autre mode préféré, on peut également utiliser une photo-activation par microscope (PPM) : On peut en envoyant des UV à travers un microscope, les projeter sur la surface PDMS de la puce microfluidique, tel que décrit par exemple dans Jun Nakanishi, Yukiko Kikuchi, Tohru Takarada, Hidekazu Nakayama, Kazuo Yamaguchi, and Mizuo Maeda. Photoactivation of a Substrate for Cell Adhesion under Standard Fluorescence Microscopes. J. AM. CHEM. SOC. 2004, 126, 16314-16315 . En plaçant un photo-masque avec les motifs désirés dans le plan focal objet du microscope, on obtient les motifs souhaités sur la surface des canaux micro-fluidiques. De plus, l'objectif permet de rétrécir considérablement la taille des motifs du photo-masque, ce qui permet de gagner en précision.

Certaines structures d'interconnexion nécessitent une résolution de l'ordre du µm. C'est pourquoi il est souhaitable d'utiliser un masque en quartz haute résolution. Afin de rentabiliser au maximum ce support, il entre également dans les objectifs de l'invention de proposer une nouvelle méthode de lithographie facilitant sa mise en œuvre. Selon cette méthode, la surface entière d'un masque 120 en quartz, par exemple tel que représenté à la figure 8, est gravée d'une multiplicité de motifs de microcanaux et de chambres cellulaires. Cet outil permet en un seul masque d'explorer des motifs qui pourraient avoir un intérêt dans l'élaboration d'un réseau contrôlé de neurones, ou permettre de tester certaines propriétés des projections axonales comme leur propriété de fasciculation/défasciculation ou leur comportement dans des canaux arrondis.

Le but de ce masque 120 est double :
- Permettre de lithographier à la fois 4 microstructures différentes de manière standardisée. Il a été conçu pour avoir un maximum de compatibilité avec les différentes géométries de macrocanaux, et peut donc être utilisé pour toutes les géométries, ce qui offre une grande souplesse d'utilisation.
- Placer le plus de motifs différents sur un même masque en quartz (qui est assez onéreux).

Une multiplicité de motifs (96 dans l'exemple donné en figure 8) peuvent être juxtaposés les uns à côté des autres (les pas ont été standardisés). Des motifs d'alignement ont été disposés en son centre. On peut donc l'utiliser pour faire les motifs de puce possédant beaucoup de compartiments, tant que la résine utilisée pour les microstructures est une résine négative. 13 formes de micro-canaux et 6 formes de compartiments cellulaires ont été gravés sur le masque 120. Chaque forme a été reproduite avec différentes tailles, portant le nombre de motifs à 96.

Des motifs multi-échelles ont par ailleurs été gravés. Ces motifs regroupent en un seul différentes tailles et formes, le but étant d'explorer un maximum de formes en un minimum d'expériences. Par exemple, pour les canaux rétrécissant, un motif multi-échelle a été conçu, qui permet de savoir en une expérience quelle taille est favorable pour un type de neurone donné.

A titre d'exemple, un total de 96 motifs différents représentés dans la figure 8, peuvent être produits à partir d'un seul masque haute résolution. La sélection entre les différents motifs peut être effectuée par un surmasque 130 à plus basse résolution présentant des fenêtres 110, comme représenté à la figure 10.

La figure 9 représente deux exemples de motifs pouvant apparaître à travers une fenêtre 110.

Le surmasque 130 peut également comporter des motifs d'alignement 135.

L'invention peut être utilisée avantageusement pour cultiver et étudier différents types de cellules, étant particulièrement intéressante pour travailler sur des neurones.

Les neurones sont des cellules qui une fois différenciées, ne se multiplient plus. Il est donc impossible de propager des cultures cellulaires de neurones. Il est par ailleurs important d'obtenir ces neurones pendant leur pic de neurogénèse : c'est le moment ou la prolifération des neurones se termine et où la différenciation est faite. Par ailleurs ils ne sont pas encore matures, c'est à dire que leurs axones et leurs dendrites n'ont pas encore poussé, et il est donc possible de les ensemencer dans les boîtes de cultures ou les puces.

Avantageusement, on utilisera dans les dispositifs selon l'invention, du matériel neuronal prélevé sur des animaux, et notamment des souris, selon des protocoles de dissection bien connus de l'homme de l'art.

Le dispositif permet également de visualiser des cellules, et de localiser des substances au sein de compartiments cellulaires spécifiques. Le dispositif se prête particulièrement bien à toutes les techniques d'immunomarquage, d'immunohistochimie, de marquage d'ADN, de marquage fluorescent, luminescent ou chemiluminescent.

Ci-dessous est donnée à titre d'exemple une liste de molécules intéressantes pour la biologie cellulaire, qui peuvent être détectées par immunomarquage fluorescent dans des dispositifs selon l'invention :
- α-tubuline : Cette sous-unité de la tubuline est présente dans toutes les microtubules des cellules.

Ce marquage permet donc de voir la totalité des cytosquelettes des cellules présentent dans le compartiment.
- β3-tubuline : Sous-unité spécifique des microtubules des axones, sauf exception.
- Map2 : Map2 est associée aux microtubules. Elle est surtout présente dans les dendrites.
- Synaptophysine : Lorsqu'un axone n'a pas de synapse, la synaptophysine est présente de façon homogène dans l'axone. Elle s'accumule dans les synapses lorsque celles-ci maturent et se connectent à un neurone.
- p-erk : erk (extra cellular regulated kynase) est une protéine kynase qui, lorsqu'elle est activée, est phosphorylée puis transloquée dans le noyau. Son activation intervient dans la cascade de transduction du signal glutamatergique des neurones striataux. On peut vérifier à l'aide de ce marquage que les neurones striataux ont bien été activés par le glutamate.

On peut également utiliser un marquage non-immunologique. Par exemple, on peut utiliser dans les dispositifs selon l'invention le DAPI ou tout autre intercalant de l'ADN, qui permet de visualiser les noyaux des cellules.

La figure 11 est une photographie d'un exemple de réalisation d'un système d'interconnexion comportant des microcanaux dont la largeur décroît d'une chambre microfluidique à l'autre. Sur la figure 11, on voit à gauche le compartiment somatique où ont été ensemencés les neurones corticaux. Les axones traversent les microcanaux 10 dont la largeur passe par exemple de 15 µm en entrée à 3µm en sortie.

On voit sur la photographie qu'à gauche, les neurones corticaux projettent des faisceaux d'axones dans la partie large des microcanaux tandis qu'à droite les faisceaux d'axones corticaux, indiqués par des flèches en pointillés ressortent du côté rétréci et se connectent aux somas et aux axones striataux repérés par des flèches en trait continu.

Sur la figure 12, on voit sur la photographie de gauche la sortie des microcanaux les plus étroits, en contraste de phase 40x, avec une culture corticale seule et sur la photographie de gauche dans le cas d'une cocultutre. Dans ce dernier cas, le réseau d'axones corticaux est beaucoup plus diffus et enchevêtré.

On a représenté à la figure 1C un exemple de dispositif similaire à ceux représentés à la figure 1B. La figure 1D est une coupe selon ID-ID de la figure 1C. Les figures IE et IF représentent les détails IE et IF des figures 1C et 1D. La figure 1H représente un détail de la figure 1G, qui est une vue en perspective du dispositif de la figure 1C.

La portion 4c peut être utile pour réduire le risque qu'une substance chimique migre d'un macrocanal 4a ou 4b vers l'autre.

Dans un exemple, la longueur du système d'interconnexion 3, mesurée perpendiculairement aux microcanaux, est par exemple comprise entre 3 mm et 5 mm, et vaut par exemple 4 mm.

La largeur d'un macrocanal 4a ou 4b est par exemple comprise entre 500 et 1500 µm et vaut par exemple 1000 µm. La largeur de la portion 4c est par exemple comprise, dans sa région intersectant les microcanaux, entre 50 et 150 µm, étant par exemple de 100 µm. La largeur d'un microcanal 10 est par exemple de 10 µm à son arrivée au départ de la portion 4c.

La hauteur des microcanaux 10 est par exemple inférieure ou égale à 5 µm, étant par exemple de 3 µm. La hauteur de la portion 4c est par exemple inférieure à 100 µm, étant par exemple de 55 µm.

### Exemples

Les dispositifs microfluidiques mis en œuvre dans les exemples ci-après peuvent être tels que décrits en référence aux Figures 1A ou 1B.

Les dispositifs utilisés peuvent être composés d'au moins deux chambres (4a et 4b) de culture séparées par des microcanaux (3) par exemple. Chaque macrocanal d'une chambre est relié aux portions élargies formant réservoir (5), qui sont par exemple perforées au travers de l'elastomère en cas de réalisation en PDMS.

Chaque dispositif est par exemple répliqué 4 fois sur le même substrat, comme illustré à la figure 1A qui représente quatre dispositifs indépendants à deux chambres chacun.

La figure 1B représente 4 dispositifs indépendants à trois chambres chacun (cas chambres étant encore parfois appelées compartiments).

Les deux (ou 3 ou plus) chambres de culture sont séparées par des systèmes d'interconnexion à microcanaux ou autres microstructures.

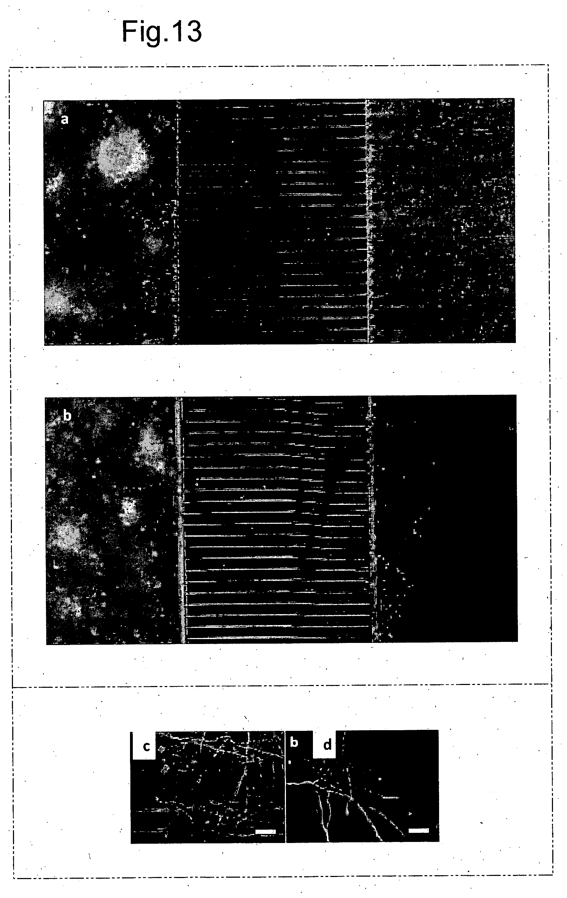

Plus précisément, on a utilisé les dispositifs suivants:
Dispositif D1 (Fig 19) :
   Le dispositif comporte 2 chambres de culture de dimensions (L x l x h en µm) 4500, 1000, 55 sont interconnectées par 167 microcanaux rétrécissant (encore appelés diodes) de 500 µm de longueur et de largeur 15 µm du coté large, 5µm du coté étroit, 3µm de hauteur. Chaque macrocanal est relié à deux réservoirs (portions élargies) de diamètre 4mm et de hauteur comprise entre 5 et 10 mm. Les chambres sont liées à des lamelles de verre de 170 µm d'épaisseur.
Dispositif D2 (Fig 13, 14, 15, 16, 17,) :
   2 chambres de culture de dimensions (L x l x h en µm) 4500, 1000, 55 sont interconnectées par 167 microcanaux rétrécissant (encore appelés diodes) de 500 µm de longueur et de largeur 15 µm du coté large, 3µm du coté étroit, 3µm de hauteur. Chaque chambre (macro canal) est reliée à deux réservoirs (portions élargies) de diamètre 4mm et de hauteur comprise entre 5 et 10 mm. Les chambres sont liées à des lamelles de verre de 170 µm d'épaisseur.
Dispositif D3 (Fig 11, 12, 18) :
   2 chambres de culture de dimensions (L x l x h en µm) 4500, 1000, 55 sont interconnectées par 167 microcanaux rétrécissant (encore appelés diodes) de 500 µm de longueur et de largeur 15 µm du coté large, 2µm du coté étroit, 3µm de hauteur. Chaque chambre (macrocanal) est reliée à deux réservoirs de diamètre 4mm et de hauteur comprise entre 5 et 10 mm. Les chambres sont liées à des lamelles de verre de 170 µm d'épaisseur.
Dispositif D4 (Fig 20, 21) :
   3 chambres de culture parallèles, à savoir 2 chambres distales de dimensions (L x l x h en µm) 4500, 1000, 55 et une chambre centrale (4500, 100, 55) sont interconnectées par 167 micro-canaux de 500 µm de longueur et de largeur 15 µm du coté large, 3µm du coté étroit, 3µm de hauteur. Chaque chambre est reliée à deux réservoirs (portions élargies) de diamètre 4mm et de hauteur comprise entre 5 et 10 mm. Les chambres sont liées à des lamelles de verre de 170 µm d'épaisseur. Certains dispositifs ont été réalisés en utilisant des micro canaux rétrécissant permettant un passage « en série » et de 500 µm de longueur, 15 µm du coté large, 3 µm coté étroit et 3µm de haut. Certains dispositifs sont réalisés avec une chambre centrale (Lxlxh en µm) 4500, 50, 3 séparée des deux autres chambres par des micro-canaux de longueur 250 µm, droits ou retrécissant (15µm x 3 µm x 3µm).
Dispositif D5 (Fig 22, 23) :
   Il s'agit de dispositifs à 3 ou 4 chambres dont la première est orientée de façon perpendiculaire aux deux autres. Les chambres sont toutes de dimensions (L x l x h en µm) 4500, 1000, 55. La première chambre (A) est reliée à la seconde (B) par une série de 100 micro-canaux de longueur comprise entre 500 et 1000 µm et de section 10µm x 10 µm x 3 µm. Ces micro-canaux rejoignent la deuxième chambre par son coté perpendiculaire. Les deuxième (B) et troisième (C) chambres sont reliées entre elles par 167 micro-canaux de 500 µm de long et de 10µm x 10 µm x 3µm de section. Certains dispositifs ont été réalisés avec des microcanaux rétrécissant de 15 µm x 3 µm x 3µm. Certains dispositifs étaient dotés de 4 chambres, la chambre (B) étant reliée sur le même principe à deux chambres perpendiculaires (A) et (A') situées de part et d'autres de la chambre (B).
Dispositif D6 (Fig 24) :
   Le dispositif comporte 3 chambres de culture parallèles, à savoir 2 chambres distales de dimensions (L x l x h en µm) 25000, 1000, 55 et une chambre centrale (25000, 3000, 55), interconnectée avec chacune des chambres distales par 930 micro-canaux de 250 µm de longueur et de section 15 µm du coté large, 5µm du coté étroit, 3µm de hauteur.

Les deux séries de micro canaux convergent vers la chambre centrale. Chaque chambre (macrocanal) est reliée à deux réservoirs (portions élargies) de diamètre 4mm et de hauteur comprise entre 5 et 10 mm. Les chambres sont liées à des lamelles de verre de 170 µm d'épaisseur.

### Exemple 1 : Masters en résine pour la fabrication de dispositifs selon l'invention.

Deux couches de résines sont utilisées afin que le master possède des structures de deux épaisseurs différentes, à savoir 3 µm et 55 µm. Les microcanaux sont réalisés avec de la résine SU8 2002 (Microchemicals) et les macrocanaux (épaisseur 55 µm) avec une résine négative laminable SY355 (Microchem).

Les deux masques de photolithographie pour la réalisation du master en résine du canal microfluidique sont dessinés à partir du logiciel QCad. Des lames de verres (1mm d'épaisseur, 52 mm de diamètre, Ediver, France) sont lavées à l'isopropanol, trempées dans un mélange piranha (50 % H₂O₂, VWR International, 50 % H₂SO₄, VWR International) pendant 30 minutes, puis séchées à 150°C pendant 2 heures. Les lames sont traitées au plasma 30s. La résine SU8 est étalée sur la lame à une vitesse déterminant son épaisseur (http ://www.microchem.com/products/pdf/SU8_2-25.pdf). Une pré-cuisson sur plaque chauffante suit l'étalement pour éliminer le solvant restant dans la résine. La résine est ensuite insolée 8 secondes aux UV (aligneur Karl Suss MJB3) à travers un support en quartz de 3mm. Une post-cuisson permet la réticulation de la résine. Le développement est conduit dans le développeur approprié à la résine jusqu'à l'apparition des motifs. Le substrat est rincé en fin de protocole. Un recuit final permet de renforcer l'adhérence de la résine au substrat et de durcir les structures en achevant la réticulation. Il faut ensuite déposer la deuxième résine. Les lames sont préchauffées à 80 °C. Le film de résine est laminé à 65°C sur les lames, insolé aux UV pendant 10 sec avec le masque pour les macro-canaux, puis développé. Un rinçage à l'isopropanol achève le procédé.

### Exemple 2 : Procédé de fabrication des dispositifs.

La fabrication des dispositifs se fait en 2 étapes.
1) Moulage du PDMS : Le moulage du dispositif se fait en versant du PDMS avec 1 :10 w/w d'agent réticulant (Sylgard 184, Dow Corning), dans un moule en PTFE contenant le master. L'ensemble est ensuite laissé à réticuler 4 heures au four à 65°C. Les trous des réservoirs sont ensuite réalisés à l'aide d'un poinçon de diamètre 4-5 mm.
2) Collage de la puce : Pour sceller le dispositif, une lame de verre est collée sur la puce en PDMS. Pour cela les 2 surfaces sont soigneusement nettoyées à l'isopropanol, puis sont traitées au plasma air (PDC-32G, Harrick) pendant 45 secondes, puis la puce et la lame de verre sont mises en contact.

### Exemple 3 : Traitement de surface

On décrit ici différents exemples de traitement de surface du substrat de dispositifs selon l'invention, (par exemple des lames de verre, afin de faciliter l'adhésion des neurones ou la progression des axones.

Dans le protocole décrit ci-dessous, la protéine utilisée est de la fibronectine rendue fluorescente par un greffage de FITC. Cependant, ces protocoles peuvent être utilisés tels quels pour fonctionnaliser les lames avec d'autres molécules intéressantes pour telle ou telle application particulière.

Préparation des lames de PDMS :
Dans un premier temps des lames de verre 4 cm de diamètre (Edriver) sont nettoyées à l'isopropanol. Puis du PDMS à 10 % en réticulant est déposé sur la lame et étalé par rotation à 1000 rpm. Les lames sont ensuite mises au four à 65°C au moins 2 heures. Les lames sont ensuite traitées avec l'un des 3 protocoles suivants :
- Les lames sont traitées au plasma 45 secondes et sont tout de suite utilisées. Elles conservent alors un caractère à la fois radicalaire et hydrophile.
- Les lames sont traitées au plasma et sont conservées au minimum 4h en contact avec de l'eau. Elles perdent ainsi leur caractère radicalaire mais conserve leur hydrophilie.
- Les lames ne sont pas traitées au plasma et ont donc une surface hydrophobe. Traitement sulfo-sanpah : Du sulfo-sanpah (Pierce) solide est dilué à 250 µg=mL dans une solution
d'HEPES (50 mM à pH 8,5). Il possède une couleur rouge vif. 200 µL de sulfo-sanpah est déposé sur la lame et insolé sous une lampe à UV jusqu'au noircissement de la solution (5 min). La lame est lavée 2 fois à l'HEPES sous agitation au shaker pendant 15 min. Une goutte de la solution de fibronectine-FITC diluée à 0.2 mg/mL dans du PBS est déposée sur la lame qui est placée dans une boîte de petri conservée humide à l'aide d'une lingette imbibée d'eau. La lame est laissée une nuit à 4°C avant d'être rincée au PBS 1X pour être utilisée.

Traitement benzophénone : Une lame est placée dans une solution de benzophénone à 10 % d'eau /acétone pendant 1 min puis rincée à l'acétone. Une goutte de solution de fibronectine-FITC diluée à 0.2 mg/mL est déposée sur la lame que l'on illumine aux UVs de la même manière que pour le sulfo-sanpah pendant 20 min. La lame est ensuite rincée au PBS.

Traitement BBTA : La solution de BBTA à 62mM est complétée avec 0,25 mg/mL de solution de fi-bronectine FITC et 0,25 mg/mL de fibronectine naturelle. La lame est exposée aux UV, puis rincée au tampon PBS

### Exemple 4 : Culture primaire et ensemencement des neurones

### Neurones en grains de cervelet (CGN)

Les cervelets sont prélevés sur des souriceaux de lignée Swiss âgés de 5 à 7 jours. Après avoir procédé à la décapitation de toute la portée, 3 incisions sont réalisées en arrière de la tête afin de dégager les hémisphères cérébelleux. Ils sont ensuite prélevés chirurgicalement, et déposés dans une boîte de pétri contenant du PBS (Gibco). Une fois les méninges retirés, les cervelets sont découpés finement, rincés 3 fois au PBS et dissociés en trypsine-EDTA (Gibco) 10 minutes à 37°C. La trypsine est ensuite inhibée par l'ajout de 10 % de SVF. Les cellules sont remises en suspension dans une solution de DMEM Glutamax I en présence de 5 U/ml de DNAse 1 (sigma), puis dissociées mécaniquement à l'aide d'une pipette de 10ml. Après 3 centrifugations de 6 minutes à 80g, le culot est resuspendu à une densité de 50 millions de cellules par ml, dans du DMEM Glutamax (Invitrogen), contenant 10% de sérum de veau foetal (Biochrom) Streptomycine/ Penicilline (Gibco), 25 mM de KCl et complémentés avec les suppléments neuronaux N2 et B27 (Gibco). Les cellules sont ensuite ensemencées dans le compartiment somatique du dispositif. 10 µL de la suspension cellulaire sont introduits dans le réservoir haut dU compartiment somatique. Les cellules s'écoulent alors rapidement dans le compartiment et après une dizaine de secondes du milieu complet sans cellules est ajouté dans l'autre réservoir de manière à stabiliser le flux créé par la différence de pression hydrostatique, et ainsi permettre aux cellules d'adhérer au support. La vitesse du flux et la qualité de l'ensemencement sont contrôlés à l'aide d'un microscope. Une dizaine de minutes après ensemencement, les 4 réservoirs (somatiques et distaux) sont remplis de milieux complets (environ 50 µL / réservoir). Entre 20 et 30000 cellules sont ainsi ensemencées dans le compartiment distal. Les puces sont placées dans une boîte de pétri contenant 2 à 3 ml d'eau de façon à éviter l'évaporation du milieu. Le milieu de culture est entièrement renouvelé tous les 2-3 jours, sur une durée qui s'étend sur 12 jours in vitro (JIV). Après deux jours de culture les neurones commencent à se différencier en émettant des neurites dans le compartiment distal. Environ 2 % des neurones ensemencés projettent leurs axones dans les micro-canaux. Après 4 à 6 jours de culture, les axones engagés dans les microcanaux rejoignent le compartiment distal, pour atteindre une longueur totale de 750 à 1200 µm après 10 jours et ne varient plus avec le temps.

Cette expérience a été réalisée avec le dispositif D1.

Culture primaire de neurones du cortex et du striatum.

Les cortex et les striata d'embryons de souris âgés de 14 jours, sont prélevés à partir de lignée swiss.

Les outils sont les mêmes que pour les CGN. Les deux hémisphères sont prélevés après avoir entaillé le tour de la boîte crânienne. Le cortex et le striatum peuvent être prélevés simultanément. Les cellules sont ensuite dissociées enzymatiquement puis mécaniquement dans du milieu L15, avant d'être ensemencées dans les dispositifs de culture selon le même protocole que celui utilisé pour les CGN. Le milieu de culture est composé de Neurobasal contenant 2mM de Glutamax-I, de la streptomycine et de la pénicilline, et enfin des suppléments de culture N2 et B27. Après 2 jours de culture les neurones corticaux commencent à se différencier en projetant des neurites dont certains commencent à entrer dans les micro-canaux. Environ 2 % des neurones corticaux ensemencés se projettent dans les microcanaux.

### Exemple 5 : protocoles d'étude par Immunofluorescence des cultures

Toutes les incubations sont effectuées à température ambiante et un flux est généré afin de pouvoir traiter l'ensemble du circuit (micro-canaux). Au temps d'analyse désiré, les cellules sont fixées au paraformaldéhide 4 % en PBS 20 min. Après rinçage au PBS, elles sont perméabilisées en triton X-100, 0,1 % pendant 10 min et saturées pendant 40 min avec une solution de PBS contenant 1%de BSA. Après rinçage, les cellules sont incubées à l'obscurité en présence de dilution d'anticorps primaires d'intérêts pendant 40 min à température ambiante. Après rinçage avec du PBS, les anticorps secondaires dirigés contre les immunoglobulines (IgG) de l'espèce ayant servi à générer le premier anticorps dilués dans une solution de DAPI, sont ajoutés pendant 25 min à l'obscurité. Après rinçage au PBS, les puces sont ensuite scellées avec du milieu de montage «fluoromount ». Les anticorps primaires dirigés contre les protéines d'intérêt suivantes ont été utilisés : _β-tubuline (monoclonal 1/300eme, Sigma), _α-tubuline (monoclonal 1/300eme, Sigma), Map 2 (polyclonal lapin, 1/300eme, Chemicon), synaptophysine (monoclonal 1/400eme, Sigma).

Les anticorps secondaires anti-espèce couplés aux Alexa 350, 488 ou 555 ont été dilué au 1/500eme. Les sondes suivantes ont été utilisées : phalloïdine couplée au cy3 (1/700eme, sigma), Mitotracker red (Molecular probes). Les acquisitions d'images sont réalisées à l'aide d'un microscope Axioberver Z1 (Zeiss) équipé d'une caméra CCD refroidie 12 bits (CoolsnapHQ2, Ropert Scientific) l'ensemble étant piloté par le logiciel open source Micro-Manager. Les Images sont traitées à l'aide du logiciel Image J.

### Exemple 6 : Culture de neurones en grains de cervelet dans un dispositif selon l'invention.

Les NGCs fraîchement dissociés sont ensemencés dans le compartiment somatique. Contrairement aux dispositifs de culture cellulaire conventionnels où toutes les cellules introduites dans un puits de culture adhèrent, le nombre de cellules ensemencées dans les circuits microfluidiques ne peut être estimé qu'après comptage au microscope du nombre de cellules ayant adhéré. Il apparaît ainsi que la densité cellulaire dans le compartiment somatique est approximativement de 20 000 à 30 000 cellules selon les expériences.

Après 2 jours in vitro (JIV), les neurones commencent à établir un réseau complexe de neurites dans le compartiment somatique dont certains commencent à pénétrer dans le premier tiers des micro-canaux. A 4 JIV, l'ensemble des 150 micro-canaux est entièrement occupé par des axones en extension dans les canaux.

Le compartiment distal est peu à peu envahi par les neurites. A partir de 7 JIV, la pousse neuritique se termine et les axones atteignent leur taille qui est de 750 à 1200 µm depuis le soma, et ne varient plus avec le temps.

Dans les systèmes de l'art antérieur, la pousse neuritique s'effectue de façon égale, selon qu'on ensemence le compartiment de droite ou le compartiment de gauche.

Un des avantages de l'invention est d'arriver à obtenir des co-cultures pour lesquelles les projections neuronales sont contrôlées (où des neurones corticaux se projettent sur des striataux par exemple). On ensemence donc un type neuronal dans la chambre de gauche, et un autre type neuronal dans le compartiment dans la chambre de droite. Pour que la projection soit contrôlée il est important que les axones puissent traverser les micro-canaux dans un sens et non dans l'autre. Dans cet exemple, on a utilisé des micro-canaux selon l'invention de forme similaire à celle représentée à la figure 2B faisant 15µm de large du côté somatique et 3 µm du côté distal pour une longueur de 500µm. Ainsi les axones du côté rétréci n'entrent pas dans les canaux, alors que les axones du côté large peuvent tout de même traverser car ils sont focalisés. Les axones ne traversent le microcanal que dans un seul sens.

Axones corticaux : Lorsque l'on ensemence des neurones corticaux du côté large (15µm), 8 à 10 axones corticaux traversent par microcanal (1000 - 1500 sur tout le système). Lorsqu'ils sont ensemencés de l'autre côté, seulement 5 à 10 axones traversent la puce dans son ensemble.

Axones striataux : Les axones corticaux entrent du grand côté (15µm) des canaux rétrécissants mais ils n'arrivent pas à franchir la partie faisant 3 µm de large. Ces dimensions de microcanal sont donc particulièrement bien adaptées à une co-culture dans laquelle on contrôle la projection des axones corticaux sur les neurones striataux, et montre que l'invention peut également être utilisée pour sélectionner la pousse d'axones en fonction du type cellulaire.

### Exemple 7 :

Application à la co-culture cortico-striatale et à la constitution de réseaux neuronaux orientés.

Principe : En ensemençant la chambre de gauche avec des neurones corticaux primaires de souris et la chambre de droite avec des neurones striataux (3 jours après), et en utilisant un milieu de culture adapté aux deux types neuronaux, une projection neuronale unidirectionnelle des neurones corticaux sur les neurones striataux a pu être observée au 6ème jour.

Cette expérience a été réalisée avec le dispositif D2.

Réseaux cortico-striatal : Des neurones corticaux et striataux sont prélevés par micro-dissection sur des embryons de souris swiss âgés de 14 jours de gestation. Toutes les étapes de la dissection sont réalisées dans du tampon PBS supplémenté de 0,1% de glucose. Les structures micro-disséquées sont digérées par incubation dans une solution de trypsine-EDTA et dissociées mécaniquement à la pipette pasteur. Après plusieurs rinçages, les suspensions mono cellulaires sont numérées. Les neurones corticaux sont re-suspendus à 40.106 cellules/ ml dans du milieu de culture DMEM, les neurones striataux à 15-20.106 cellules/ml. Les neurones corticaux et striataux sont ensuite ensemencés dans leur chambre de culture respective, les neurones corticaux du coté large des microcanaux du système d'interconnexion. Les réservoirs de culture sont alors emplis de milieux de culture composés de DMEM contenant du glutamax, de la pénicilline et streptomycine et des suppléments neuronaux B27 et N2. Les dispositifs microfluidiques ensemencés sont alors placés à l'incubateur à 37°C. Le milieu de culture est renouvelé tous les 3 jours.

On a vu qu'avec un dispositif selon l'invention présentant une dissymétrie de largeur entre les deux côtés du système d'interconnexion, on facilitait la co-culture corticaux-striatale. Il peut être utile dans cette expérience de co-culture de vérifier que la projection des synapses des neurones corticaux sur les neurones striataux est bien fonctionnelle.

Orientation du faisceau d'axones corticaux : La croissance des faisceaux d'axones corticaux est très sensible à la présence des neurones striataux : ils s'orientent très nettement vers eux.

Maturation des synapses : En utilisant un marquage synaptophysine, on vérifie que la proximité des neurones striataux entraîne bien le bourgeonnement des synapses des axones corticaux ayant traversé le micro-canal. On peut voir la maturation des synapses des neurones corticaux dans un cas mais pas dans l'autre.

### Exemple 8 : Filtration de neurones d'hippocampe et de cortex

La figure 13 comporte des photographies illustrant la filtration des axones de neurones de l'hippocampe par un dispositif selon l'invention.

Cette expérience a été réalisée avec le dispositif D2.

Les figures a et b illustrent la filtration de neurones corticaux par des microcanaux disymétriques, encore appelés diodes axonales. Image a du haut: les neurones corticaux sont ensemencés du coté large des microcanaux. Les noyaux neuronaux sont marqués en bleu (colorant nucléaire, Dapi) et les prolongements axonaux en vert (beta tubuline). On peut noter l'invasion par les axones de la chambre de droite. Image b du bas : les neurones corticaux sont ensemencés du côté étroit des microcanaux, seuls quelques axones traversent. La photographie c représente des axones de l'hypocampe sortant du coté étroit des microcanaux lorsque les cellules sont ensemencées du coté large. Cinq sorties de microcanaux sont apparentes. La photographie d représente des axones de l'hypocampe sortant du coté large des microcanaux lorsque les neurones sont ensemencés du coté étroit (cinq sorties de microcanaux sont visibles, seule une est occupée). (Barres d'échelle 25µm).

**Exemple 9** : Reconstitution de réseaux de neurones dans un dispositif selon l'invention et visualisation des connections synaptiques dans des réseaux cortico-striatum et des réseaux cortico-hippocampe. Evaluation de la fonctionnalité synaptique.

Cette expérience a été réalisée avec le dispositif D2.

Réseaux cortico-striatal : Des neurones corticaux et striataux sont prélevés par micro-dissection sur des embryons de souris swiss âgés de 14 jours de gestation. Toutes les étapes de la dissection sont réalisées dans du tampon PBS supplémenté de 0,1% de glucose. Les structures micro-disséquées sont digérées par incubation dans une solution de trypsine-EDTA et dissociées mécaniquement à la pipette pasteur. Après plusieurs rinçages, les suspensions mono cellulaires sont numérées. Les neurones corticaux sont re-suspendus à 40.106 cellules/ ml dans du milieu de culture DMEM, les neurones striataux à 15-20.106 cellules/ml. Les neurones corticaux et striataux sont ensuite ensemencés dans leur chambre de culture respective, les neurones corticaux du coté large des microcanaux. Les réservoirs de cultures sont alors emplis de milieux de culture composée de DMEM contenant du glutamax, de la pénicilline et streptomycine et des suppléments neuronaux B27 et N2. Les dispositifs microfluidiques ensemencés sont alors placés à l'incubateur à 37°C. Le milieu de culture est renouvelé tous les 3 jours.

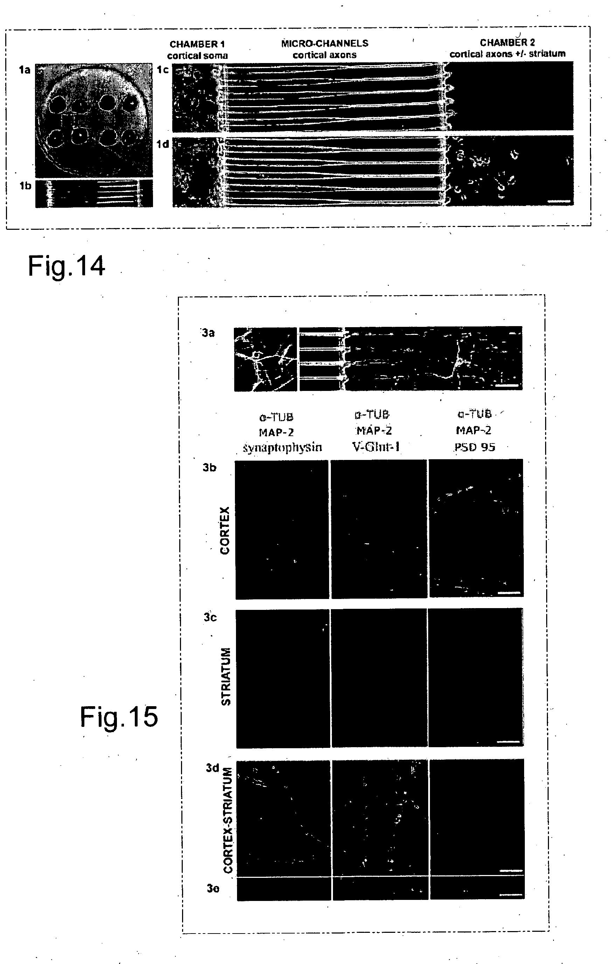

La photographie la de la figure 14 représente des dispositifs microfluidiques de culture de neurones selon l'invention, comportant deux champs de culture individuels interconnectés par une série de microcanaux asymétriques, dont quelques uns sont visibles sur la photographie 1b.

Ces microcanaux empêchent les corps des cellules de pénétrer tout en permettant la croissance des axones dans une direction, comme décrit plus haut.

Les photographies c et d sont des images par contraste de phase combinées avec épifluorescence, d'un réseau neuronal reconstruit polarisé.

Pour visualiser les axones individuels des neurones corticaux, les cellules ont été transduits par le vecteur viral M-cherry-Sindbis.

La photographie la représente les neurones corticaux développant leurs axones dans la seconde chambre et la photographie 1d les neurones striataux ensemencés dans la seconde chambre et recevant des neurones corticaux provenant de la première chambre.

On peut noter que les neurones striataux ne sont pas contaminés par le virus, ce qui est représentatif d'une compartimentation efficace des deux chambres de culture.
Barre d'échelle : 50 µm.

### Exemple 10 : Co-culture microfluidique cortico-striatal avec maturation synaptique accrue.

La photographie 3a de la figure 15 représente des neurones striataux, après mise en culture pendant trois jours dans la seconde chambre d'un dispositif selon l'invention, et des neurones corticaux ensemencés dans la première chambre.

Des neurons corticaux et striataux sont prélevés par micro-dissection sur des embryons de souris swiss âgés de 14 jours de gestation. Toutes les étapes de la dissection sont réalisées dans du tampon PBS supplémenté de 0,1% de glucose. Les structures micro-disséquées sont digérées par incubation dans une solution de trypsine-EDTA et dissociées mécaniquement à la pipette pasteur. Après plusieurs rinçages les suspensions mono cellulaires sont numérées. Les neurones corticaux sont re-suspendus à 40.106 cellules/ ml dans du milieu de culture DMEM, les neurones striataux à 15-20.106 cellules/ml. Les neurones corticaux et striataux sont ensuite ensemencés dans leur chambre de culture respective, les neurones corticaux du coté large des microcanaux. Les réservoirs de cultures sont alors emplis de milieux de culture composée de DMEM contenant du glutamax, de la pénicilline et streptomycine et des suppléments neuronaux B27 et N2. Les dispositifs microfluidiques ensemencés sont alors placés à l'incubateur à 37°C. Le milieu de culture est renouvelé tous les 3 jours.

Après dix jours de co-culture, les neurones ont été marqués au niveau de la tubuline (vert), de MAP 2(rouge) et en DAPI (bleu).

Les neurones corticaux ont envoyé leurs axones à travers les microcanaux, sont sortis des microcanaux et se sont étendus à travers la deuxième chambre de culture, où ils se sont connectés aux neurones striataux.

Les photographies 3b et 3c représentent une coloration synaptique d'un réseau cortico-striatal MAP E (bleu) - tubuline (vert) et un marquage pré-ou post-synaptique (Vglut-1, synpatophysine ou PSD95, rouge) de
(b) neurones corticaux, ou
(c) neurones striataux, et
(d) neurones striataux, en contact avec les fibres corticales.

On peut remarquer sur la photographie 3b que les neurones corticaux présentent des amas faibles et dispersés de synaptophysine et de Vglut-1 sur leurs axones, témoignage d'une maturation partielle des terminaux présynaptiques. La photographie 3c représente des neurones striataux cultivés séparément qui présentent des amas épars de synaptophysine et PSD95, signalant un état intermédiaire de différenciation des neurones striataux.

Comme attendu, les neurones striataux cultivés séparément n'ont pas exprimé Vglut-1.

La photographie 3d montre que les connexions développées par le contact entre les fibres corticales et les dendrites striatales conduisent à un marquage accru avec un regroupement de ces trois marqueurs synaptiques.

On peut noter une augmentation de la coloration et de la formation d'amas de synaptophysine, de Vglut-1 et de PSD95, plus particulièrement où les axones corticaux et les neurones striataux semblent être en contact les uns avec les autres, témoignant d'une relocalisation des protéines synaptiques aux contacts cortico-striataux.

La photographie 3e représente à échelle agrandie un marqueur synaptique (rouge) vers les dendrites striatables (bleu).

Barre d'échelle pour la photographie 3a: 50 µm et pour les photographies 3b à 3e : 10 µm.

### Exemple 11: Reconstitution d'un réseau cortico-hippocampe dans un dispositif microfluidique selon l'invention.

Cette expérience a été réalisée avec le dispositif D2.

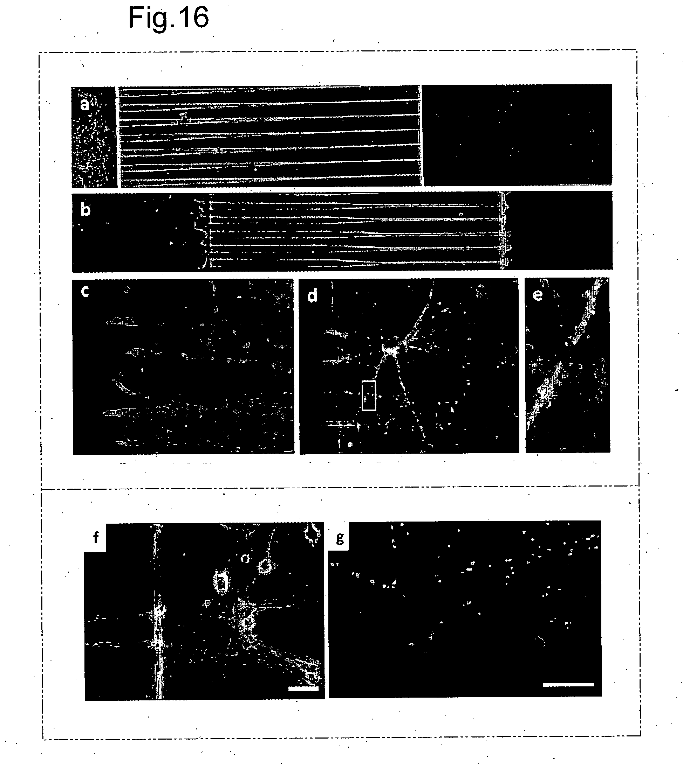

La photographie a, en contraste de phase, de la figure 16 représente un dispositif ensemencé avec des cortex seuls et la photographie b en contraste de phase également un dispositif ensemencé avec des cortex dans la chambre de gauche s'étendant sur des neurones d'hippocampe. Les photographies c, d et e correspondent à des marquages immunofluorescents. La photographie c représente des axones de cortex ensemencés seuls, la photographie d des axones de cortex s'étendant sur un neurone d'hippocampe. Les photographies e et f représentent un faisceau d'axones corticaux (a-tubuline en vert) mettant en évidence un bourgeonnement synaptique (regroupement de synaptophysine en rouge) à proximité de la dendrite (protéine associée à la microtubule MAP2 en bleu) du neurone d'hippocampe.

La photographie f représente un réseau cortico-gyrus denté, avec un faisceau de fibres corticales (tubuline, en vert) contactant des neurones du gyrus denté (Prox-1, rouge). L'image g est similaire, avec un marquage de la formation de synapses cortico hippocampique (a synaptohysine, rouge) le long de dendrites de neurones du gyrus denté (MAP2, bleu). Grossissement X400(a) X630 (b). Barres d'échelle 20 µm.

### Exemple 12 : Translocation des protéines pré-synatpiques corticales au contact des dendrites des neurones d'hippocampe.

La photographie a de la figure 17 correspond à un marquage de la synaptophysine (rouge) et de la tubuline axonale (vert) de neurones corticaux ensemencés seuls.

Cette expérience a été réalisée avec le dispositif D2.

Des neurones corticaux sont prélevés par micro-dissection sur des embryons de souris swiss âgés de 14 jours de gestation. Les neurones d'hippocampes sur des embryons de souris gés de 16 à 19 jours. Toutes les étapes de la dissection sont réalisées dans du tampon GBSS supplémenté de 0,1% de glucose. Les structures micro-disséquées sont digérées par incubation dans une solution de papaine-EDTA et dissociées mécaniquement à la pipette pasteur. Après plusieurs rinçages, les suspensions mono cellulaires sont numérées. Les neurones corticaux sont re-suspendus à 40.106 cellules/ ml dans du milieu de culture DMEM, les neurones d'hippocampe à 15.106 cellules/ml. Les neurones corticaux et d'hippocampe sont ensuite ensemencés dans leur chambre de culture respective, les neurones corticaux du coté large des microcanaux. Les réservoirs de cultures sont alors emplis de milieux de culture composée de DMEM contenant du glutamax, de la pénicilline et streptomycine et des suppléments neuronaux B27 et N2. Les dispositifs microfluidiques ensemencés sont alors placés à l'incubateur à 37°C. Le milieu de culture est renouvelé tous les 3 jours.
Photographie (b) : Neurones d'hippocampes ensemencés seuls, sans afférence corticale : marquages des dendrites (MAP2, bleu), des axones (tubuline, vert) et de la synaptophysine (rouge).
Images (c, d) : Faisceaux de fibres corticales (vert) et de la synaptophysine d'hippocampe (MAP2, bleu).
On peut noter l'intense marquage des protéines présynaptiques le long des dendrites des neurones d'hippocampe (synaptophysine, rouge).
Image (e) : Grossissement d'une dendrite d'hippocampe. On peut noter que la synaptophysine se trouve aux points de jonction entre les fibres corticales (tubuline en vert) et la dendrite du neurone d'hippocampe (MAP2 en bleu). (a, b, c, d) Barres d'échelle 25 d'échelle 10 µm.

### Exemple 13 :

La figure 18 représente des chambres microfluidiques séparées par des microcanaux asymétriques.

Cette expérience a été réalisée avec le dispositif D3.

Les neurones corticaux sont ensemencés à gauche et les neurones striataux à droite. Après sept jours *in vitro,* les axones corticaux établissent des connexions avec les neurones striataux (β3 tubuline en vert, neurones striataux en rouge (MAP2).

La dépolarisation avec KCl déclenche une phosphorylation de la kinase erk striatale (en rouge), un procédé dépendant de la neurotransmission par glutamate (comme évalué avec inhibition avec MK801)

### Exemple 14 : Evaluation de l'effet axo-protecteur de molécules pharmacologiques d'intérêt thérapeutique.

Des neurones du cervelet ensemencés dans des dispositifs microfluidiques selon l'invention et traités par un inducteur d'apoptose (Staurosporine 1µM, STS) au niveau somatique. Cela induit une dégénérescence axonale dans la chambre distale (Phi). Des composés bloquant l'apoptose (zVAD) ou les protéases de type calpaine (Cal-Inh) sont appliqués spécifiquement sur les axones (et pas sur les somas). L'effet « axo protecteur » est quantifié sur le graphe de droite de la figure 19.

On utilise des dispositifs D1.

Des neurones (ici du cervelet) sont ensemencés et leurs axones traversent les microcanaux asymétriques pour rejoindre la seconde chambre (ou la 3eme si le dispositif est tri-comartimenté).

Un stress (ici la staurosporine, un agent pro-apoptotique) est appliqué sur le soma des neurones. Il induit une dégénérescence des axones en 24h. L'application concomitante, sur l'axone, d'un inhibiteur de l'apoptose (zVAD, ou des calpaines (Cal-Inh) prévient cette dégénérescence. On peut donc évaluer l'effet de molécules « axo-protectrice). C

La figure 19 illustre l'enclenchement d'une dégénérescence axonale induite par l'application d'un stress pro-apoptotique appliqué sur le compartiment somato-dentritique de neurones. Ceci provoque la propagation d'un signal destructif dans l'axone qui dégénère alors précocement, avant le soma cellulaire. L'application de molécules bloquant l'apoptose (zVAD) ou les calpaines (iCal), ou de NAD, retardent cette dégénérescence axonale.

### Exemple 15 : Dispositif à trois chambres permettant l'application localisée de molécules d'interet sur une portion d'axone.

Le dispositif microfluidique représenté sur l'image 4a de la figure 20 est similaire à celui décrit précédemment en référence aux figures 1C à 1H et comporte trois chambres. Il s'agit du dispositif D4. Ceci permet d'appliquer des molécules d'intérêt sur la portion centrale des axones, par exemple un détergent (TritonX100, 0,1%), appliqué pendant 1 min. Cela provoque une section immédiate de la portion centrale des axones, comme illustré par la photographie 4b. Dans cet exemple, alors que les axones de cortex sectionnés dans leur portion centrale sont encore intact après une heure de section, ils dégénèrent après 4h. Ceci constitue un modèle de dégénérescence wallerienne (dégénérescence de la portion distale des axones). Le potentiel de composés protecteurs de cet effet peuvent être évalué en les appliquant spécifiquement dans une ou plusieurs des chambres du dispositif. Le graphe 4 de la figure 20 quantifie l'effet axoprotecteur du NAD.

### Exemple 16 : Modélisation d'un phénomène de dégénérescence synaptique sur un réseau cortico-striatal, et évaluation de molécules d'intérêt contre la dégénérescence synaptique. NAD+ retarde la déconnexion.

Les photographies a et b de la figure 21 représentent des chambres corticales, centrales et striatales de réseaux reconstruits cortico-striataux dans des dispositifs à trois chambres microfluidiques.

Les neurones ont été marqués sur MAP2 (bleu) 3β-tubuline (vert), et Vglut-1 (rouge).

La photographie a représente des neurones corticaux ayant envoyé les axones à travers les microcanaux et la chambre centrale pour atteindre et connecter des neurones striataux.

La photographie b montre qu'une heure après l'axotomie, plus aucun axone n'est présent dans la chambre centrale, mais que les axones à l'intérieur des microcanaux et à l'intérieur de la chambre prostriatale sont demeurés intacts.

La photographie c est représentative de structures cortico-présynaptiques (Vglut1, rouge) fixées à des dentrites striatables (MAP2, bleu) après 1, 2, 4, 6 heures d'une axotomie ou culture contrôle ou prétraitée avec NAD.

On peut remarquer la diminution de la coloration Vglut-1 après une durée aussi courte qu'une seule fois après l'axotomie.

La photographie d illustre la quantification d'une structure corticale présynaptique fixée à des dentrites striatales positives à MAP 2 après l'axotomie corticale.

Echelle sur les photographies 5a et b : 50 µm et la photographie 5c : 5µm.

Cette expérience a été réalisée avec le dispositif D4. L'expérience, réalisée sur un réseau cortico-striatal, permet d'illustrer que l'on peut objectiver un phénomène de dégénérescence synaptique après application d'un stress au niveau cortical (ici axotomie) (disparition des marquages Vglu-1 qui sont spécifique des structures pre-synaptiques corticales). L'exemple 15 montre que l'on peut objectiver un phénomène de dégénérescence axonale. L'expérience de l'exemple 16 montre aussi que l'on peut évaluer le rôle de molécules protégeant / stabilisant les synapses après enclenchement d'un stress distant. Les figures 20 et 21 illustrent les conséquences de l'axotomie d'axones corticaux d'un réseau cortico-striatal. Cette axotomie provoque la propagation d'un signal aboutissant à dégénérescence rapide du compartiment pre synaptique (figure 21) de la jonction cortico-striatale, puis à la dégénérescence des axones corticaux (figure 20). L'ajout de la molécule NAD dans le compartiment striatal au moment de l'axotomie retarde, la dégénérescence pre-synaptique, et la dégénérescence des axones corticaux transectés.

### Exemple 18 : Reconstitution d'un réseau à 3 types de neurones dans un dispositif tri-chambre à système d'interconnexions orientés perpendiculairement.

On utilise dans cet exemple un dispositif similaire à celui précédemment décrit en référence à la figure 22.

On utilise le dispositif D5, représenté sur les figures 22 et 23, composé de 3 chambres (ABC) de culture séparées par des microcanaux asymétriques (longueurs variant entre 500 et 1000 µm entre A et B, et 500µm entre B et C).

La particularité de ce dispositif est que les chambres (macrocanaux) A et B sont perpendiculaires. Les neurones (ici de cortex) ensemencés dans la chambre A projettent leurs axones dans la chambre B et continuent tout droit.

Ils sont donc orientés de façon perpendiculaire aux micro-canaux reliant les chambres B et C. Ceci évite qu'ils ne traversent la chambre B de part en part pour rejoindre la chambre C.

Des neurones corticaux sont prélevés par micro-dissection sur des embryons de souris swiss âgés de 14 jours de gestation. Les neurones de CA sur des embryons de souris gés de 16 jours. Et les neurones de DG sur des souriceaux nouveaux nés agés de 5 jours postnatal. Toutes les étapes de la dissection sont réalisées dans du tampon GBSS supplémenté de 0,1% de glucose. Les structures micro-disséquées sont digérées par incubation dans une solution de papaine-EDTA et dissociées mécaniquement à la pipette pasteur. Apres plusieurs rinçages les suspensions mono cellulaires sont numérées. Les neurones corticaux sont re-suspendus à 40.106 cellules/ ml dans du milieu de culture DMEM, les neurones d'hippocampe à 15.106 cellules/ml. Les neurones corticaux et CA ou DG sont ensuite ensemencés dans leur chambre de culture respective, les neurones corticaux du coté large des diodes. Les réservoirs de cultures sont alors emplis de milieux de culture composée de DMEM contenant du glutamax, de la pénicilline et streptomycine et des suppléments neuronaux B27 et N2. Les dispositifs microfluidiques ensemencés sont alors placés à l'incubateur à 37°C. Le milieu de culture est renouvelé tous les 3 jours.

L'image de la figure 23 représente des neurones corticaux ensemencés dans la chambre proximale supérieure (en bleu). Les axones corticaux traversent les micro-canaux et rejoignent la seconde chambre (en vert). Aucun neurone cible n'est introduit dans les autres chambres. On peut noter les projections rectilignes des axones corticaux sur l'image b. Des neurones du gyrus denté DG (dont les noyaux sont marqués en rouge) sont introduits dans la chambre intermédiaire (en vert sur l'image f). On peut noter que les axones corticaux en vert convergent vers les neurones du DG. On peut noter également les projections des neurones du DG vers la chambre distale (en rouge sur (f)).

L'image (c) représente un réseau à 3 neurones reconstitué sur le même principe que pour les images (a) et (b).Les neurones corticaux sont ensemencés dans la chambre proximale supérieure (non visible sur cette image). Les axones corticaux qui sortent des micro-canaux contactent les neurones du DG dans la seconde chambre, encadrée en haut à gauche et en zoom sur l'image (d). Les neurones du DG envoient leurs axones dans la 3eme chambre et contactent les neurones de l'hypocampe, encadré au milieu en bas et visible sur l'image (e). Barres d'échelles 250µm sauf pour l'image (f) 5mm.

### Exemple 19 : Détection de protéines au sein d'extraits axonaux de neurones corticaux développés dans une chambre microfluidique.

Le schéma a de la figure 24 illustre un dispositif microfluidique comportant trois chambres interconnectées. Les neurones sont ensemencés dans les deux chambres latérales et leurs axones respectifs atteignent la chambre centrale. Les chambres microfluidiques sont isolées fluidiquement par la pression hydrostatique. Juste avant la lyse axonale, les chambres de culture cellulaire contenant le soma des neurones sont remplies de gel diagarose. Ceci assure une isolation permanente du compartiment somatique. Le tampon de lyse cellulaire circule dans le compartiment central et permet la récupération spécifique du matériel axonal sans contamination somatique.

La photographie b illustre une coloration en or colloïdal d'extrait somatique et axonal unique.

Le dispositif utilisé est le dispositif D6.

Des neurones de cortex de souris sont ensemencés dans les deux canaux extérieurs et les axones de ces neurones rejoignent la chambre centrale. Ceci permet de majorer la quantité de matériel axonal dans la chambre centrale. A l'issue de la période de culture cellulaire, un tampon de lyse est perfusé dans le compartiment (axonal ou somatique) que l'on souhaite spécifiquement prélever. Cet échantillon peut ensuite être soumis à des analyses ultérieures, ici électrophorèse en gel d'acrylamide et révélation par la technique de Western Blot.

Préalablement à l'étape de perfusion du tampon de lyse, un gel (ex ici : agarose à faible point de fusion) peut être introduit dans les compartiments que l'on ne souhaite pas prélever. Ceci permet de « figer » ces compartiments et assure une parfaite isolation fluidique lorsqu'on perfuse le tampon de lyse dans le compartiment à prélever.

La photographie c illustre un « Western blot » typique à la fois des compartiments axonaux et somatiques pour MAP2 (dendritique) et beta 3 tubuline (axonal) et actine.

### Exemple 20

Dans cet exemple, on utilise un dispositif microfluidique de culture de neurones présentant trois chambres, tel que représenté aux figures 1C à 1H.

Les microcanaux sont utilisés pour diriger la pousse des axones et compartimenter les cultures. Ces circuits neuronaux tri-compartimentés ont été fabriqués à l'aide de méthodes de photolithographies, comme décrit précédemment. L'architecture de ces circuits microfluidiques comprend une chambre (macrocanal) somatique et une chambre (macrocanal) axonale de 1mm de long chacune et une chambre centrale de 100 µm de long. Les chambres sont connectées entre elles par une série de micro-canaux parallèles de 10 µm de large, 3 µm d'épaisseur et 450 µm de long, et espacés de 20 µm les uns des autres, comme sur les figures 1C à 1H. L'épaisseur des compartiments somatique, central et axonal est de 55 µm. Pour fabriquer des masters présentant des canaux de deux hauteurs différentes, deux couches de résine photosensible sont utilisées. Les masters de silicium sont d'abord chauffés à 150°C et activé par un traitement plasma pendant 30s. La première couche de résine photosensible SU8-2002 (Microchem, Newton, USA) est spin-coatée sur la plaque de silicium à 2000rpm puis on réalise un recuit à 65°C pendant 2 min et un recuit à 95°C pendant 4 min. La résine est ensuite exposée à la lumière UV à travers un masque optique où sont dessinés les micro-canaux. Après un nouveau recuit, les micro-canaux de 2-3 µm d'épaisseurs sont développés avec le développeur à SU8 (Microchem). Un film de résine SY355 (Elga Europe, Milano, Italy) d'une épaisseur de 55 µm est ensuite laminé à 80°C sur le master présentant déjà les micro-canaux. Un second masque présentant la géométrie des trois compartiments est ensuite aligné sur les micro-canaux préexistant et exposé aux rayons UV. Le tout est ensuite cuit à 120°C pendant 5 min et la seconde couche est développée dans le développeur BMR (Elga Europe). Le tout est enfin rincé à l'isopropanol. On obtient ainsi un master constitué de compartiments et de micro-canaux en relief en résine qui reposent sur une lame de silicium. Chaque master présente quatre circuits microfluidiques identiques comportant chambres et micro-canaux. On moule ensuite un élastomère, le polydiméthylsiloxane (PDMS), à l'aide de ces masters. Le PDMS (Dow Corning, Midland, MI) non réticulé est coulé sur les masters puis mis à réticuler au four à 70°C pendant trois heures. On obtient après cuisson une couche de 4-5 mm d'épaisseur de PDMS mis en forme. Des trous de 4 mm sont ensuite percés dans cette couche de PDMS, à l'aide de punchs de biopsie, et constituent des réservoirs de milieu de culture. Les trous ainsi formés sont légèrement plus larges que les réservoirs moulés dans le PDMS à l'aide du master ; ils les englobent complètement et sont directement reliés aux différents compartiments du circuit. Chaque moulage de PDMS est ensuite lié irréversiblement à une lamelle de verre (Menzel Glass, Braunschwein, Germany) via une activation des surfaces (face moulée du PDMS et une face de la lamelle de verre) avec un générateur de plasma à air (Diener Electronic, Nagold, Germany) suivie d'une mise en contact manuelle des deux surfaces activées. Les circuits ainsi formés sont stérilisés par exposition aux rayonnements UV et traités pendant une nuit à l'aide d'une solution de Poly-D-Lysine (Sigma, Saint Louis, USA) à 10 µg/mL afin que les surfaces intérieures du circuit restent hydrophiles.

### Cultures de Neurones et traitements.

Les cortex d'embryons de souris de 14 jours sont disséqués et dissociés comme cela a déjà été décrit ailleurs, par exemple Eur J Neurosci 2001 Jun; 13(11): 2037-46 William S *et al.* Les neurones sont ensemencés dans la chambre (encore appelée compartiment) somatique des circuits à une densité de 10⁵ cellules/cm². De cette façon on ensemence à peu près 4.10⁴ neurones dans la chambre somatique. Le milieu de culture est constitué de DMEM contenant 4.5 g/L D-glucose et pyruvate (Invitrogen, Carlsbad, USA) additionné de 10% de SVF (sérum de veau foetal ; PAA Laboratories, Pasching, Germany) et de 2% de B27 (Invitrogen). Le milieu est remplacé tous les trois jours jusqu'au début de l'expérience et les cultures sont conservées dans un incubateur à 37°C et 5% CO₂. Les expériences sont menées après 7 à 8 jours de culture in vitro. L'inhibiteur de Calpain N-Acetyl-Leu-Leu-Nle-CHO (ALLN ; Calbiochem), l'inhibiteur général de caspase Z-V-A-D-Fluoromethyl cetone (z-VAD ; R1D Systems, Minneapolis, USA), et la β-Nicotinamide adenine dinucléotide hydratée (βNAD ; Sigma) sont introduits dans le milieu de culture au début des expériences.

### Procédure d'axotomie.

Pour réaliser l'axotomie, un flux de 15 µL de milieu contenant 0.1% de saponine est produit pendant 2 minutes dans la chambre centrale. Pour éviter que le flux de détergent entre dans les micro-canaux, une surpression est maintenue dans les chambres somatiques et axonale tout au long de l'expérience. A près 2 minutes de flux, le milieu contenant la saponine est retiré et le macrocanal central est rincé avec du milieu de culture pendant 5 min. On ajoute ensuite du milieu frais dans ce canal.

### Expériences de flux et de diffusion.

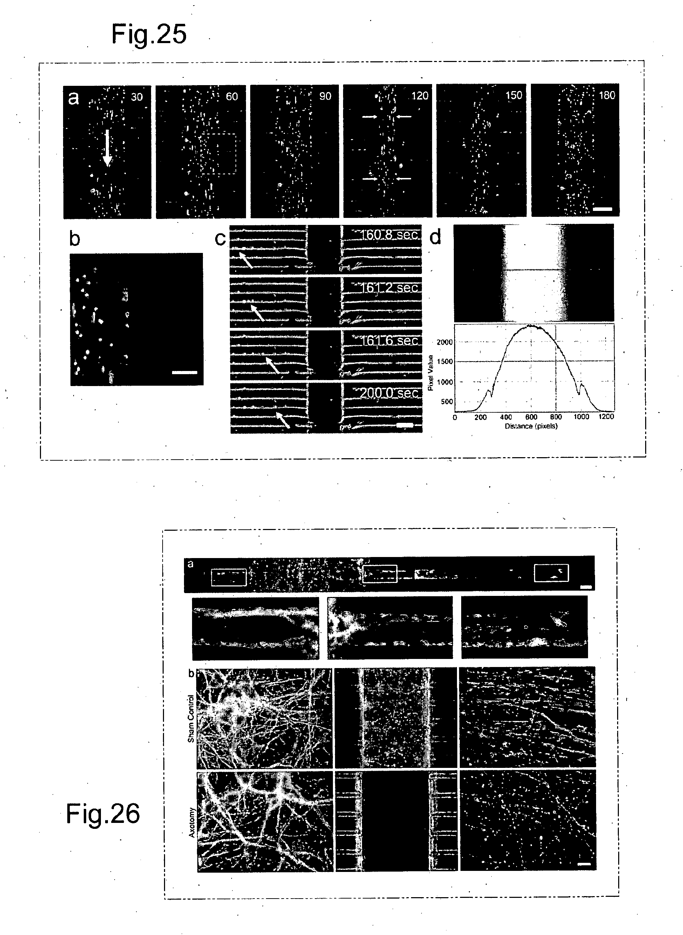

Une série d'expériences qualitatives de mesure de flux et de diffusion ont été réalisées pour vérifier (i) que l'axotomie est localisée dans la chambre centrale et (ii) que les traitements à long terme restent confinés dans les compartiments dans lesquels ils sont adressés. Pour simuler la procédure d'axotomie une expérience a été menée en réalisant un écoulement de microsphères fluorescentes de 1 µm de diamètre (Invitrogen) en suspension dans un mélange de PBS et de saponine et en suivant l'écoulement de ces billes par vidéo microscopie. D'autres part, des anticorps anti-lapin Immunoglobuline G conjugués à un Alexa Fluor 555 (IgG ; 1 :100, Invitrogen) ont été utilisés comme traceur de diffusion pour simuler l'effet de la diffusion de particules tant pendant l'axotomie que pendant les traitements à long terme. La relation entre l'intensité de fluorescence dans le circuit et la concentration du marqueur a été établie en capturant des images des compartiments microfluidiques préalablement remplis avec des solutions contenant le marqueur à des concentrations connues. Une relation linéaire (R²>0.99) a été mise en évidence entre l'intensité du signal de fluorescence et la concentration du traceur. Dans chaque expérience trois circuits ont été analysés et, dans chacun de ces circuits, les images de trois régions différentes de chaque compartiment ont été réalisées à différents temps pour quantifier la diffusion du marqueur fluorescent (i) en partant du compartiment central et en allant vers le compartiment somatique pour les expériences mimant l'axotomie, (ii) depuis le compartiment axonal en allant vers les compartiments central puis somatique pour les expériences mimant les traitements à long terme. Le signal non-spécifique a été obtenu en réalisant une acquisition avec le filtre DAPI et a été soustrait au signal de l'Alexa Fluor 555 pour éliminer la variation de l'intensité du signal due aux variations locales de l'intensité d'illumination. L'axotomie a été mimée en ajoutant une solution 100% du marqueur à la solution de détergent et en mettant les mêmes volumes de solutions dans les réservoirs que pour une véritable axotomie. Les images de la figure 25 ont été réalisées respectivement 2, 5 et 10 min après l'ajout de la solution de détergent contenant le marqueur dans le réservoir du compartiment central. Les expériences mimant la diffusion de molécules à long terme ont été réalisées en introduisant la solution contenant le marqueur dans la chambre axonale et en maintenant l'équilibre des pressions dans le circuit en plaçant des volumes identiques de liquide dans tous les réservoirs. Les images ont été prises 1, 2, 4 et 8 heures après l'ajout du marquer dans la chambre axonale.

### Immunocytochimie.

Après un temps de culture donné (éventuellement en fonction du moment de l'expérience d'axotomie), les cultures de neurones sont fixées avec une solution à 4% de Paraformaldéhyde (Sigma) pour subir une analyse par immunocytochimie. Les circuits sont colorés par immunofluorescence avec un anticorps anti-MAP2 (lapin IgG, 1 :3000 ; Chemicon, Temecula, USA), et un anticorps monoclonal anti-β-III-tubuline (souris IgG, 1 :3000 ; Sigma) pour le marquage des protéines du cytosquelette. Les circuits sont colorés en fluorescence avec du Hoechst 33342 (1 :25000 ; Sigma) pour marqué le noyau. Dans une expérience séparée, les circuits ont également été colorés en immunofluorescence avec un anticorps anti-glial fibrillary acidic protein (GFAP, lapin IgG, 1 : 5000 ; DakoCytomation, Glostrup, Denmark) qui permet de marquer les astrocytes. Une partie des cultures a été soumise à une fixation et une extraction. Cette procédure permet de supprimer la tubuline libre dans le cytoplasme tout en fixant les microtubules. Ces cultures ont été colorées en immunofluorescence avec un anticorps monoclonal anti-α-tubuline conjugué au fluorophore FITC (Sigma) et colorées par de la phalloidine liée à un Alexa Fluor 555 (Invitrogen) pour révéler les microtubules et le cytosquelette d'actine, respectivement. Les images ont été acquises à l'aide d'un microscope inversé à épi-fluorescence (Zeiss, Goettingen, Germany) équipé d'une caméra CoolSnap HQ2 (Roper Scientific, Ottobrunn, Germany) et en utilisant le logiciel d'acquisition micro-manager (http://www.micro-manager.org).

### Quantification

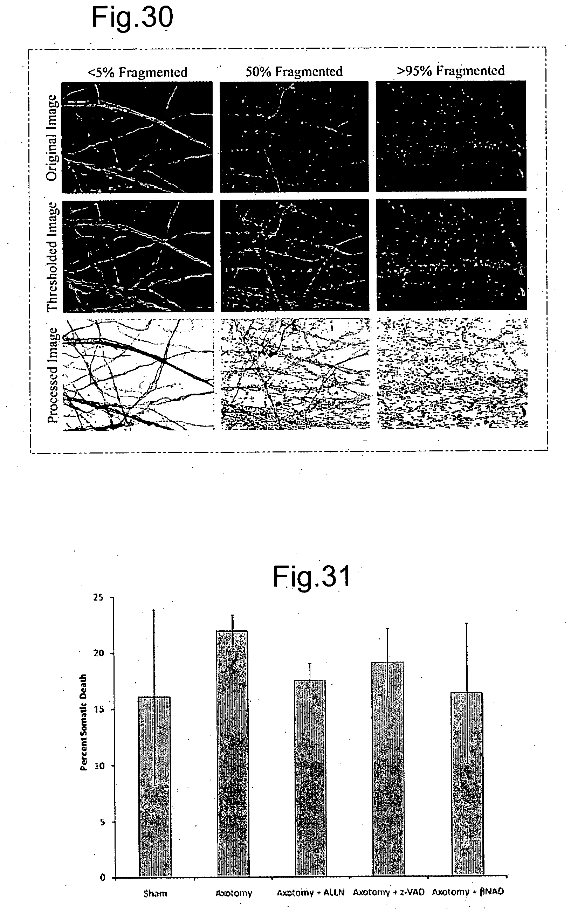

La dégénérescence axonale est estimée en comparant le type de marquage de la tubuline d'axones fragmentés ou d'axones intacts. La bonne survie, en culture, des neurones corticaux est dépendante de la densité d'ensemencement initiale qui doit être forte. En conséquence, un grand nombre d'axones corticaux envahissent les chambres centrales et distales. Par ailleurs, les axones corticaux présentant de nombreuses ramifications, l'analyse par immunohistochimie de la tubuline axonale dans les chambres axonales est donc complexe, et les axones sont difficilement individualisable. La proportion d'axone entrant dans la chambre centrale et ressortant dans la chambre axonale distale a été estimée en calculant un ratio d'intensité de fluorescence globale du marquage de la b3 tubuline, de surfaces identiques capturées dans la chambre centrale et axonale distale. Pour mesurer l'index de fragmentation axonale, on peut précéder par une méthode d'analyse d'images automatisée. Les images d'axones viables ou fragmentés, marqués pour la 3 tubuline, sont capturées par microscopie à epifluorescence. Les axones fragmentés présentent un marquage ponctiforme et discret de la tubuline, en revanche les axones intacts présentent un marquage linéaire. A l'aide d'une macro-commande développée sous le logiciel Image J, et en utilisant séquentiellement, un algorithme de segmentation de type Otsu (voir IEEE Trans Systems, Man and Cyber Metics 1979 ; 9 :92-69) et un algorithme d'analyse particulaire, les segments axonaux présentant un index de circularité >= à 0,9 sont considéré comme des segments d'axones fragmentés. Pour chaque image, la surface totale de ce type de marquage (circularité >=0,9) est déterminée et rapportée à la surface totale du marquage de la tubuline (indépendemment de la topologie du marquage). Ce ratio est appelé « index de fragmentation » et est un indicateur de la moyenne de l'état de fragmentation des axones étudies. La figure 30 montre la relation entre l'indice de fragmentation et le % d'axone fragmentés, 0,005, 0,083 et 0,157 correspondant respectivement à <5%, 50% et >95 % d'axones fragmentés. La survie des soma neuronaux est évaluée i) par analyse de la condensation de la chromatine nucléaire révélée par un marquage DAPI et ii) par l'analyse de la morphologie dendritique après marquage MAP2. Au moins 5 images par condition ont été analysées (chaque condition étant tripliquée), les expériences ayant été réalisées au moins 3 fois de manière indépendante.

### Analyse statistique

Les données sont exprimées sous forme de moyennes +/- l'écart à la moyenne (sem). N représente le nombre de circuits individuels. L'analyse statistique est réalisée à l'aide d'une analyse de variance unilatérale (ANOVA), suivie d'un test de Tukey.

### Résultats

### Contrôle des écoulements fluides dans le compartiment d'axotomie.

Afin de caractériser l'écoulement de fluides dans la chambre centrale du dispositif on peut réaliser des expériences d'écoulement d'une suspension de billes fluorescentes dans une dilution de détergent Pour obtenir un flux contrôlé dans un compartiment, un gradient de pression est réalisé en plaçant des volumes différents de liquide dans les réservoirs connectés à la chambre correspondante. Ainsi, on peut suivre par video microscopie l'écoulement de la suspension de billes dans le canal central lorsque les réservoirs d'ajout (inlet) et d'extrait (outlet) sont remplis de respectivement 15 et 0 µl, alors que les deux chambres connexes sont pressurisées avec 40µl de PBS dans chacun de leurs réservoirs (tous les réservoirs ayant les mêmes dimensions). Les images acquises pendant les 180 premières secondes montrent que la solution de détergent n'entre pas dans les micro-canaux jouxtant la chambre centrale. Dans la partie basse (encore appelé macrocanal) du canal central, les billes sont contraintes dans la partie centrale de la chambre, montrant ainsi l'existence d'un flux des chambres sur-pressurisées vers la chambre centrale ; il y a donc focalisation de la suspension de détergent dans le canal central (fig 25a). En utilisant les mêmes conditions fluidiques, l'expérience contraire consistant à emplir les deux chambres distales d'une suspension de billes, alors qu'un écoulement de PBS est généré dans la chambre centrale, montre que dans ces conditions un flux des chambres distales vers la chambre central est objectivable par le mouvement de billes dans les micro-canaux (fig 25b et 25c). Par ailleurs, afin de déterminer plus précisément les paramètres des gradients générés dans cette configuration d'écoulement, et de simuler une expérience d'axotomie, une dilution d'immunoglobuline fluorescente à été utilisée comme traceur. Aucune diffusion de l'Ig fluorescente n'a pu être détectée de la chambre centrale vers l'intérieur des micro-canaux. Dans la partie basse de la chambre centrale, après 2 minutes d'écoulement, la dispersion du traceur dans la chambre (le long de la section de la chambre) s'établit suivant une gaussienne dont le pic est au centre de la chambre, pour atteindre une valeur nulle aux bordures. Ceci montre que l'immunoglobuline fluorescente est bien confinée dans le canal central par le flux en provenance des 2 chambres distales sur pressurisées (Fig 25d et table 1 ci-après). L'expérience inverse à été réalisée en ajoutant le traceur dans les deux chambres distales ; et montre l'écoulement des chambres distales vers la chambre centrale.

### Table 1

Expérience de diffusion d'un traceur pendant l'axotomie.

La concentration de traceur est reliée linéairement à l'intensité signal fluorescent (R² > 0.99). Les valeurs sont données en % de la concentration maximum et représente la moyenne de 3 expériences indépendantes.

| | 2 min | 5 min | 10 min |
|---|---|---|---|
| Compartiment Somatique | 0.02 ± 0.29 | 0.18 ± 0.30 | 0.29 ± 0.31 |
| Compartiment Central | 68.4 ± 9.8 | 58.1 ± 3.9 | 44.9 ± 5.8 |

Des expériences supplémentaires ont été réalisées pour étudier la diffusion de traceurs d'une chambre vers l'autre lors d'une compartimentalisation au long court. Des volumes identiques de liquide ont été introduits dans tous les réservoirs (aucun flux généré) et le traceur fluorescent à été ajouté dans l'une des chambre distale. 2 heures après l'ajout du traceur, environ 15% du traceur est retrouvé dans la chambre centrale, et rien dans la chambre distale opposée, montrant ainsi que la chambre centrale sert de « tampon » permettant d'assurer une parfaite compartimentalisation des deux chambres distales, la chambre centrale jouant dans cette configuration un rôle de « syphon ». Les valeurs sont indiquées dans le tableau 2.

**Table 2 Expérience de diffusion du traceur à long terme. La concentration de traceur est reliée linéairement à l'intensité signal fluorescent (R² > 0.99). Les valeurs sont données en % de la concentration maximum et représente la moyenne de 3 expériences indépendantes.**

| | 0h | 1h | 2h | 4h | 8h |
|---|---|---|---|---|---|
| Compartiment Axonal | 83.2 ± 7.91 | 72.8 ± 0.52 | 66.7 ± 0.55 | 76.1 ± 4.79 | 69.4 ± 10.7 |
| Compartiment Central | 1.41 ± 1.36 | 4.23 ± 4.25 | 13.3 ± 0.70 | 13.5 ± 0.11 | 13.1 ± 0.60 |
| Compartiment Somatique | 0.26 ± 0.13 | 0.34 ± 0.13 | 0.32 ± 0.02 | 0.37 ± 0.15 | 0.26 ± 0.00 |

Dans leur ensemble, ces expériences objet de l'exemple 20 montrent i) qu'il est possible de procéder à un écoulement contrôlé et confiné dans la chambre centrale sans aucune diffusion de cette chambre vers les deux chambres distales adjacentes ; ii) que la diffusion à long terme de molécule introduites dans une chambre distale vers la seconde chambre distale est complètement abolie par la présence d'une chambre centrale, ce qui permet de s'assurer de la parfaite isolation fluidique d'une chambre axonale vers une chambre somatique (ou l'inverse).

### Différenciation des neurones de cortex

Les neurones issus de cortex de souris embryonnaires sont ensemencés à relativement haute densité dans la chambre « somatique ». Les axones d'environs 3 à 5% (proche de la zone des micro-canaux) des neurones ensemencés pénètrent dans les 167 micro-canaux qui rejoignent la chambre centrale. Le phenotypage des cellules ensemencées après 8 jours de culture montre que la culture est composée de 90 à 90% de neurones (MAP2 positifs), 5 à 8 % d'astrocytes (GFAP positifs) et de cellules microgliales (1%) et fibroblastiques (1%), Dans les conditions d'ensemencement pratiquées environs 10 à 15 axones entrent par micro-canal. L'analyse par immunocytochime de la tubuline axonale montre que 38,8% +/-3,4 % (n=11) des axones ayant envahit la chambre centrale (de 100µm de large) rejoignent effectivement la seconde chambre distale après 8 jours de culture (La proportion étant d'environ 60% lorsque la largeur de la chambre centrale est de 50µm). Après 10 jours de culture la longeure moyenne des axonex corticaux ayant traversé la chambre centrale et rejoint la seconde chambre distale est de 1.5 à 2 mm (Fig 26). Les axones sont confinés en faisceaux dans les micro-canaux, et ont tendance à defasciculer en sortie de micro-canaux **(****Fig 26a****).** Le compartiment central du dispositif peut donc etre utilisé pour appliquer localement un traitement, sur la portion médiane des axones.

### L'axotomie des neurones de cortex induit une coalescence des cônes de croissance et un phénomène de « dégénérescence wallerienne ».

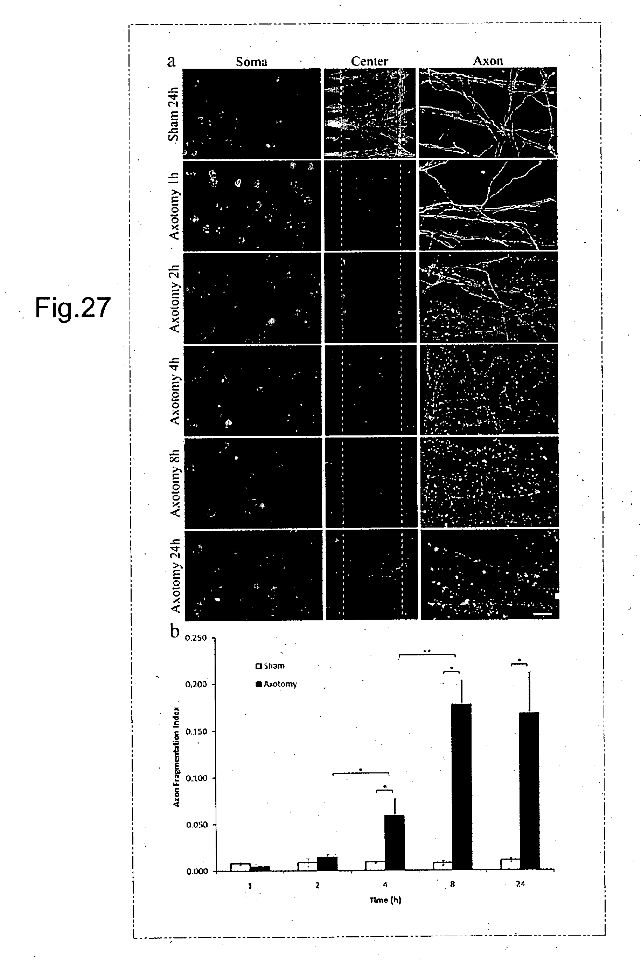

L'axotomie des neurones corticaux est réalisée par la création d'un flux (dans les conditions décrites dans le paragraphe 1) pendant 2 minutes dans le compartiment central, de milieu de culture contenant 0,1% de saponine. Le flux de détergent provoque une destruction immédiate des segments d'axones contenu dans le compartiment central (Fig 26b) provoquant ainsi une transsection située à environ 500 à 600 µm du soma neuronal. L'axotomie provoque une dégénérescence progressive de la portion distale des axones aboutissant à la formation précoce de ballonnement des segments axonaux suivi de la fragmentation du réseau de microtubules (Fig 27a). La fragmentation axonale a été quantifiée à différents temps post-axotomie et indique que l'index de fragmentation qui diffère significativement des contrôles non traités après 4h. (Les contrôles « sham » ont subit les mêmes conditions de flux (en omettant la saponine) que les axones traités à la saponine). La fragmentation augmente significativement entre tous les temps d'analyse pour atteindre un plateau, correspondant à la destruction totale de tous les axones après 8h **(****Fig 27b****).**

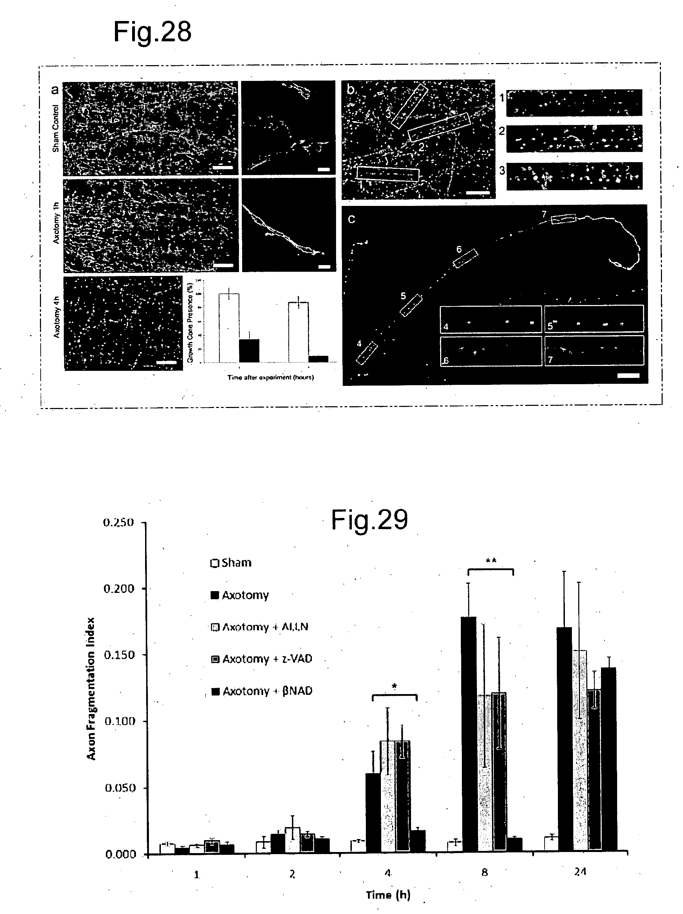

La présence de cônes de croissance à l'extrémité distale des axones corticaux est indicative de la bonne croissance axonale, le neurone cherchant sa cible. Les cônes de croissance, qui sont pauvres en tubuline, présentent une morphologie dynamique caractéristique ou les filaments d'actine forment des filopodes et des lamellipodes (voir J. Neurobiol. 2004 ; 58(1) : 92-102). Alors que dans les neurones contrôles de très nombreux cônes de croissance sont objectivables par un marquage de l'actine, 1heure âpres axotomie, alors que le réseau de microtubule est encore intact, les axones transectés présentent des cônes de croissance effondrés à leur pointe (Fig 28a). Ceci indique que rapidement après axotomie, un signal destructif s'est propagé jusqu'à l'extrémité distale des axones.

In vivo, l'axotomie des neurones périphériques a été montrée comme étant un phénomène asynchrone. A un temps donné les axones transsectés présentant des morphologies allant de la structure intacte à la fragmentation complète, la propagation antérograde de la dégénérescence ayant été suspectée. Dans notre modèle expérimental dans les phases précoce de l'axotomie (<4h) on peut observer des axones intacts, ballonnés ou fragmentés, suggérant l'enclenchement d'un phénomène légèrement asynchrone **(****Fig 28b****).** L'analyse des segments uniques d'axones transectés après 6h, montre qu'un même axone peut présenter des signes de fragmentation dans les région proximales (proches de micro-canaux), de ballonnement dans la partie médiane, et de segments intacts dans leur partie complètement distale. Ceci suggère un phénomène de propagation antérograde d'une vague de dégénérescence vers la pointe de l'axone (Fig 28c).

### La dégénérescence distale est ralentie par le bNAD mais non par l'inhibition des caspases ou des calpaines.

L'activation précoce des calpaines a été décrite âpres axotomie de neurones du système nerveux périphérique. En revanche, bien qu'elles soient activées dans les somas des neurones axotomisés, l'inhibition des caspases ne prévient pas de la dégénérescence des axones coupés. On peut évaluer l'implication des calpaines et des caspases dans la dégénérescence axonale induite par l'axotomie des neurones corticaux comme suit. L'application sur les segments distaux des axones, au moment de l'axotomie, de 50µM d'ALLN, un inhibiteur des calpaines ou de 50µM de z-VAD un inhibiteur général des caspases ne retarde pas la survenue de la fragmentation des axones, ni n'en modifie les paramètres. (Fig 29). Une absence d'effet protecteur est aussi observée lorsque ces molécules sont appliquées 24h avant l'axotomie (non montré). En revanche, lorsque les neurones sont pré traités 24h avant l'axotomie par 5mM de la molécule bNAD, les signes de dégénérescence des axones sont retardés. Alors que tous les axones contrôles sont fragmentés, 8h après axotomie les axones coupés ne présentent aucun signe de dégénérescence. Cette protection disparait 24 heures après l'axotomie **(****Fig 29****).**

La figure 31 indique la proportion de morts somatiques, obtenues en divisant le nombre de corps cellulaires avec marquage par colorant Hoechst par le nombre total de corps. Aucun changement significatif de mort somatique n'a été observé 24 heures après l'axotomie, avec ou sans traitement, comparé aux contrôles témoins.

L'exemple 20 ci-dessus peut être réalisé avec un système d'interconnexion avec des micro canaux de largeur constante ou non.

L'invention a ainsi encore pour objet, selon un autre de ses aspects, un procédé d'axotomie utilisant un dispositif microfluidique à au moins trois chambres, par exemple tel que représenté **à la** **figure 1B** **ou** **1C à 1H****,** avec des microcanaux ayant une largeur constante ou non, dans lequel un composé est injecté dans la chambre centrale pour agir sur au moins un axone s'étendant dans les microcanaux, afin de réaliser l'axotomie. Les chambres distales peuvent être en surpression par rapport à la chambre distale.

L'invention n'est pas limitée aux exemples décrits.

L'expression "comportant un" doit se comprendre comme étant synonyme de "comportant au moins un".

## Revendications

1. Dispositif de culture cellulaire, notamment de cellules neuronales, comportant :
- un support définissant une première chambre microfluidique (2a) destinée à être ensemencée par une première culture cellulaire, et au moins une deuxième chambre microfluidique (2b), et
- un système d'interconnexion fluidique (3) reliant les première et deuxième chambres microfluidiques (2a, 2b) et permettant à des extensions cellulaires, notamment des axones, de s'étendre d'une chambre microfluidique vers l'autre chambre microfluidique, dans lequel la première chambre microfluidique et la deuxième chambre microfluidique comprennent chacune un macrocanal (4a, 4b) relié à ses extrémités à des portions plus larges formant des réservoirs (5), le système d'interconnexion fluidique (3) comprenant au moins un microcanal (10) qui est relié à ses extrémités au macrocanal de la première chambre microfluidique (4a) et au macrocanal de la deuxième chambre microfluidique (4b),
**caractérisé en ce que :**
- la largeur dudit au moins un microcanal se réduit en progressant d'une chambre microfluidique vers l'autre, et
- le système d'interconnexion est réalisé de manière à privilégier la progression d'au moins un premier type d'extensions cellulaires par rapport à au moins un second type d'extensions cellulaires, lesdits premier et second types d'extensions cellulaires différant soit par la chambre microfluidique dont ils proviennent, soit par le type cellulaire dont ils sont l'extension.

2. Dispositif de culture cellulaire selon la revendication **1, caractérisé en ce que** les macrocanaux (4a, 4b) des première et deuxième chambres microfluidiques sont parallèles.

3. Dispositif de culture cellulaire selon l'une quelconque des revendications précédentes, **caractérisé en ce que** le dispositif comporte une troisième chambre microfluidique (4c) communiquant avec le système d'interconnexion fluidique (3).

4. Dispositif de culture cellulaire selon l'une quelconque des revendications précédentes, **caractérisé en ce que** le système d'interconnexion (3) comporte un microcanal (10) dont au moins une portion présente une forme trapézoïdale lorsqu'observé de dessus.

5. Dispositif de culture cellulaire selon l'une quelconque des revendications précédentes, **caractérisé en ce que** le système d'interconnexion (3) comporte un canal non rectiligne.

6. Dispositif de culture cellulaire selon l'une quelconque des revendications précédentes, **caractérisé en ce que** le système d'interconnexion (3) comporte au moins une portion dont la surface a été traitée chimiquement ou biochimiquement de façon à avoir une affinité pour au moins un type de cellule ou un type de comportement cellulaire.

7. Dispositif de culture cellulaire selon la revendication **6, caractérisé en ce que** ledit traitement chimique comporte l'exposition à au moins un type de molécule sélectionné parmi la fibronectine, les cadhérines, le collagène, la laminine, la polylysine, les molécules comportant des groupements succinimides, le (N-Sulfosuccinimidyl-6-[4'-azido-2'-nitrophenylamino]hexanoate), les molécules chimiques réactives photo-activables.

8. Dispositif de culture cellulaire selon l'une quelconque des revendications précédentes, **caractérisé en ce que** le système d'interconnexion (3) comporte des microcanaux (10) d'épaisseur comprise entre 1 et 5 µm, préférentiellement entre 2 et 4 µm.

9. Dispositif de culture cellulaire selon l'une quelconque des revendications précédentes, **caractérisé en ce que** ledit dispositif comporte au moins une chambre microfluidique monocellulaire (80), dimensionnée pour ne contenir que le soma d'une seule cellule, cette chambre microfluidique monocellulaire communiquant avec le système d'interconnexion et avec l'une des première et deuxième chambres microfluidiques.

10. Dispositif de culture cellulaire selon l'une quelconque des revendications précédentes, **caractérisé en ce que** ledit dispositif comporte au moins une microélectrode, de préférence un réseau de microélectrodes, reliée à un appareil de mesure permettant d'étudier des processus électrophysiologiques au sein d'au moins une cellule présente dans ledit dispositif.

11. Procédé de culture cellulaire, notamment de cellules neuronales, dans lequel on ensemence avec des cellules au moins une chambre microfluidique (2a, 2b) d'un dispositif de culture cellulaire selon l'une quelconque des revendications **1** à **10,** lesdites cellules pouvant, lors de leur multiplication et/ou de leur différenciation, donner lieu à des extensions cellulaires.

12. Utilisation du dispositif de culture cellulaire selon l'une quelconque des revendications **1** à **10** pour
i. une compartimentalisation neuronale efficace, l'imposition d'une directionnalité à la croissance axonale, la sélection entre différents types d'axones, et/ou la création de réseaux de cellules impliquant différents types de cellules contenus dans des chambres différentes, et présentant des connexions dirigées par l'intermédiaire des extensions cellulaires ; ou
ii. la reconstruction orientée de réseaux neuronaux du plus simple au plus complexe, impliquant éventuellement différents sous-types neuronaux, et/ou une combinaison de neurones et de cellules non-neuronales, et/ou différents types d'interactions axonales et/ou dendritiques entre neurones ; ou
iii. reconstruire et étudier les interactions entre cellules neuronales et cellules non-neuronales; ou
iv. adresser des compartiments neuronaux spécifiques avec des réactifs et/ou des médicaments, de collecter pour analyse des biomarqueurs provenant de certains de ces compartiments.

## Patentansprüche

1. Vorrichtung für Zellkultur, vornehmlich für neuronale Zellen, umfassend:
- einen Träger, eine erste mikrofluidische Kammer (2a) definierend, dafür bestimmt, von einer ersten Zellkultur besiedelt zu werden, und wenigstens eine zweite mikrofluidische Kammer (2b), und
- ein System zur fluidischen Vernetzung (3), die erste und zweite mikrofluidische Kammer (2a, 2b) miteinander verbindend und es den beiden zellulären Erweiterungen, vornehmlich Axonen, erlaubend, sich von einer mikrofluidischen Kammer zu der anderen mikrofluidischen Kammer zu erweitern, in welchem die erste mikrofluidische Kammer und die zweite mikrofluidische Kammer jeweils einen Makrokanal (4a, 4b) umfassen, an seinen Extremitäten verbunden mit breiteren Abschnitten, Behälter (5) bildend, wobei das System zur fluidischen Vernetzung (3) wenigstens einen Mikrokanal (10) umfasst, der an seinen Extremitäten mit dem Makrokanal der ersten mikrofluidischen Kammer (4a) und mit dem Makrokanal der zweiten mikrofluidischen Kammer (4b) verbunden ist,
**dadurch gekennzeichnet, dass:**
- sich die Breite des wenigstens eine Mikrokanals, von einer mikrofluidischen Kammer zu der anderen fortschreitend, verringert, und
- das Verbindungssystem solcherart beschaffen ist, dass die Progression von wenigstens einem ersten Typ von zellulären Erweiterungen in Bezug auf wenigstens einen zweiten Typ von zellulären Erweiterungen bevorzugt wird, wobei sich der erste und der zweite Typ von zellulären Erweiterungen entweder hinsichtlich der mikrofluidischen Kammer, von der sie stammen, oder hinsichtlich des Zelltyps, dessen Erweiterung sie sind, unterscheiden.

2. Vorrichtung für Zellkultur nach Anspruch **1, dadurch gekennzeichnet, dass** die Makrokanäle (4a, 4b) der ersten und zweiten mikrofluidischen Kammer parallel sind.

3. Vorrichtung für Zellkultur nach einem der vorhergehenden Ansprüche, **dadurch gekennzeichnet, dass** die Vorrichtung eine dritte mikrofluidische Kammer (4c) umfasst, mit dem System zur fluidischen Vernetzung (3) kommunizierend.

4. Vorrichtung für Zellkultur nach einem der vorhergehenden Ansprüche, **dadurch gekennzeichnet, dass** das Vernetzungssystem (3) einen Mikrokanal (10) umfasst, von dem wenigstens ein Abschnitt bei einer Betrachtung von oben eine trapezförmige Form aufweist.

5. Vorrichtung für Zellkultur nach einem der vorhergehenden Ansprüche, **dadurch gekennzeichnet, dass** das Vernetzungssystem (3) einen nicht geradlinigen Kanal umfasst.

6. Vorrichtung für Zellkultur nach einem der vorhergehenden Ansprüche, **dadurch gekennzeichnet, dass** das Verbindungssystem (3) wenigstens einen Abschnitt umfasst, dessen Oberfläche chemisch oder biochemisch behandelt wurde, um eine Affinität für wenigstens einen Zelltyp oder ein Zellverhalten zu erhalten.

7. Vorrichtung für Zellkultur nach Anspruch **6, dadurch gekennzeichnet, dass** die chemische Behandlung die Exposition gegenüber wenigstens einem Molekültyp umfasst, ausgewählt aus Fibronektin, Cadherinen, Collagenen, Laminin, Polylysin, Succinimidgruppen umfassenden Molekülen, (N-Sulfosuccinimidyl-6-[4'-Azido-2'-Nitrophenylamino]-Hexanoat), chemischen lichtaktivierbaren reaktiven Molekülen.

8. Vorrichtung für Zellkultur nach einem der vorhergehenden Ansprüche, **dadurch gekennzeichnet, dass** das Verbindungssystem (3) Mikrokanäle (10) von einer Dicke zwischen 1 und 5 µm umfasst, bevorzugt zwischen 2 und 4 µm.

9. Vorrichtung für Zellkultur nach einem der vorhergehenden Ansprüche, **dadurch gekennzeichnet, dass** die Vorrichtung wenigstens eine monozelluläre mikrofluidische Kammer (80) umfasst, dimensioniert, um nur das Soma einer einzigen Zelle zu enthalten, wobei diese mikrofluidische monozelluläre Kammer mit dem Verbindungssystem und mit einer von der ersten und der zweiten mikrofluidischen Kammer kommuniziert.

10. Vorrichtung für Zellkultur nach einem der vorhergehenden Ansprüche, **dadurch gekennzeichnet, dass** die Vorrichtung wenigstens eine Mikroelektrode umfasst, bevorzugt ein Mikroelektrodennetz, verbunden mit einem Messgerät, das Studieren der elektrophysiologischen Prozesse innerhalb der wenigstens einen in der Vorrichtung vorhandenen Zelle ermöglichend.

11. Verfahren für Zellkultur, vornehmlich für neuronale Zellen, in welchem wenigstens eine mikrofluidische Kammer (2a, 2b) einer Vorrichtung für Zellkultur nach einem der Ansprüche **1** bis **10** besiedelt wird, wobei die Zellen während ihrer Vermehrung und/oder ihrer Differenzierung zu zellulären Erweiterungen führen können.

12. Verwendung der Vorrichtung für Zellkultur nach einem der Ansprüche **1** bis **10** für
i. eine wirksame neuronale Kompartimentierung, die Auferlegung einer Richtwirkung für das axonale Wachstum, die Auswahl zwischen verschiedenen Axontypen und/oder die Erzeugung von Zellnetzen, verschiedene in den verschiedenen Kammern enthaltene Zelltypen implizierend, und mittels zellulärer Erweiterungen gesteuerte Verbindungen aufweisend; oder
ii. die ausgerichtete Rekonstruktion von neuronalen Netzen vom einfachsten zum komplexesten, eventuelle verschiedene neuronale Untertypen implizierend, und/oder eine Kombination von Neuronen und nicht-neuronalen Zellen, und/oder verschiedene Arten von axonalen und/oder dendritischen Interaktionen zwischen Neuronen; oder
iii. Rekonstruieren und Studieren der Interaktionen zwischen neuronalen Zellen und nicht-neuronalen Zellen; oder
iv. Adressieren von spezifischen neuronalen Kompartimenten mit Reagenzien und/oder Medikamenten, und Sammeln zur Analyse von aus bestimmten dieser Kompartimente stammenden Biomarkern.

## Claims

1. A device for cell culture, in particular of neuronal cells, comprising:
- a support defining a first microfluidic chamber (2a) intended to be seeded by a first cell culture, and at least one second microfluidic chamber (2b), and
- a fluidic interconnection system (3) connecting the first and second microfluidic chambers (2a, 2b) and enabling cell extensions, in particular axons, to extend from one microfluidic chamber towards the other microfluidic chamber, in which the first microfluidic chamber and the second microfluidic chamber each comprise a macrochannel (4a, 4b) connected at its ends to larger portions forming reservoirs (5), the fluidic interconnection system (3) comprising at least one microchannel (10) which is connected at its ends to the macrochannel of the first microfluidic chamber (4a) and to the macrochannel of the second microfluidic chamber (4b),
**characterised in that:**
- the width of said at least one microchannel becomes smaller as it progresses from one microfluidic chamber towards the other, and
- the interconnection system is produced so as to promote the progression of at least one first type of cell extensions compared with at least one second type of cell extensions, said first and second types of cell extensions differing either by virtue of the microfluidic chamber from which they originate, or by the virtue of the cell type of which they are the extension.

2. The device for cell culture according to claim **1, characterised in that** the macrochannels (4a, 4b) of the first and second microfluidic chambers are parallel.

3. The device for cell culture according to any one of the preceding claims, **characterised in that** the device comprises a third microfluidic chamber (4c) communicating with the fluidic interconnection system (3).

4. The device for cell culture according to any one of the preceding claims, **characterised in that** the interconnection system (3) comprises a microchannel (10) of which at least one portion has a trapezoidal shape when observed from above.

5. The device for cell culture according to any one of the preceding claims, **characterised in that** the interconnection system (3) comprises a nonrectilinear channel.

6. The device for cell culture according to any one of the preceding claims, **characterised in that** the interconnection system (3) comprises at least one portion of which the surface has been chemically or biochemically treated so as to have an affinity for at least one type of cell or one type of cell behavior.

7. The device for cell culture according to claim **6, characterised in that** the said chemical treatment comprises the exposure to at least one type of molecule selected from fibronectin, cadherins, collagen, laminin, polylysine, molecules comprising succinimide groups, N-sulfosuccinimidyl 6-[4'-azido-2'-nitrophenylamino]hexanoate, photoactivatable reactive chemical molecules.

8. The device for cell culture according to any one of the preceding claims, **characterised in that** the interconnection system (3) comprises microchannels (10) of thickness between 1 and 5 µm, preferably between 2 and 4 µm.

9. The device for cell culture according to any one of the preceding claims, **characterised in that** the said device comprises at least one single-cell microfluidic chamber (80), proportioned so as to contain only the soma of a single cell, this single-cell microfluidic chamber communicating with the interconnection system and with one of the first and second microfluidic chambers.

10. The device for cell culture according to any one of the preceding claims, **characterised in that** the said device comprises at least one microelectrode, preferably a network of microelectrodes, connected to a measuring instrument for studying electrophysiological processes in at least one cell present in the said device.

11. A method for cell culture, in particular of neuronal cells, in which at least one microfluidic chamber (2a, 2b) of a device for cell culture according to any one of claims **1** to **10** is seeded with cells, said cells being able to, during their multiplication and/or differentiation, give rise to cell extensions.

12. Use of the device for cell culture according to any one of claims **1** to **10**
i. for effective neuronal compartmentalization, the imposition of directionality on axonal growth, the selection between various types of axons, and/or the creation of networks of cells involving various types of cells contained in different chambers, and having directed connections by means of the cell extensions; or
ii. for oriented reconstruction of the simplest to the most complex neuronal networks, optionally involving various neuronal subtypes, and/or a combination of neurons and of non-neuronal cells, and/or various types of axonal and/or dendritic interactions between neurons; or
iii. to reconstruct and to study the interactions between neuronal cells and non-neuronal cells; or
iv. to target specific neuronal compartments with reagents and/or medicaments, and to collect biomarkers originating from some of these compartments.
